# EUROPÄISCHE PATENTSCHRIFT

(11) **EP 1 276 760 B1**
(45) Veröffentlichungstag und Bekanntmachung des Hinweises auf die Patenterteilung: **02.11.2006**
(21) Anmeldenummer: 01927897.7
(22) Anmeldetag: 07.04.2001
(51) Int. Cl.: C07K 14/00, C12Q 1/68, C07F 9/40

(54) **POLYAMIDNUKLEINSÄURE-DERIVATE, MITTEL UND VERFAHREN ZUR IHRER HERSTELLUNG**
POLYAMIDE NUCLEIC ACID DERIVATIVES, AGENTS AND METHODS FOR PRODUCING THEM
DERIVES D'ACIDE NUCLEIQUE POLYAMIDE, AGENTS ET PROCEDES PERMETTANT DE LES PREPARER

(30) Priorität: 18.04.2000 DE 10019135
(43) Veröffentlichungstag der Anmeldung: 22.01.2003
(73) Patentinhaber: Sanofi-Aventis Deutschland GmbH, 65929 Frankfurt am Main (DE)
(72) Erfinder: UHLMANN, Eugen, 61479 Glashütten (DE); BREIPOHL, Gerhard, 60529 Frankfurt (DE); WILL, David, William, 65830 Kriftel (DE)
(86) Internationale Anmeldenummer: PCT/EP2001/004030
(87) Internationale Veröffentlichungsnummer: WO 2001/079216

(56) Entgegenhaltungen:
- EP-A- 0 672 677
- WO-A-96/11205
- WO-A-99/33867
- WO-A-99/37670
- DE-A- 19 508 923
- DE-A- 19 640 974
- US-A- 5 874 553

### SEQUENZ PROTOKOLL

<110> Aventis Pharma Deutschland GmbH
<120> Polyamidnukleinsäure-Derivate, Mittel und Verfahren zu ihrer Herstellung
<130> AVE D-2000/A023
<140>
   <141>
<150> 10019135.5
   <151> 2000-04-18
<160> 53
<170> PatentIn Ver. 2.1
<210> 1
   <211> 21
   <212> DNA
   <213> Künstliche Sequenz
<220>
   <223> Beschreibung der künstlichen Sequenz:Oligonukleotide
o <220>
   <221> misc_binding
   <222> (1)..(21)
<400> 1
   gcgtttgctc ttcttcttgc g 21
<210> 2
   <211> 20
   <212> DNA
   <213> Künstliche Sequenz
<220>
   <223> Beschreibung der künstlichen Sequenz:Oligonukleotide
<220>
   <221> misc_binding
   <222> (1)..(20)
<400> 2
   acacccaatt ctgaaaatgg 20
<210> 3
   <211> 20
   <212> DNA
   <213> Künstliche Sequenz
<220>
   <223> Beschreibung der künstlichen Sequenz:Oligonukleotide
<220>
   <221> misc_binding
   <222> (1)..(20)
<400> 3
   aggtccctgt tcgggcgcca 20
<210> 4
   <211> 20
   <212> DNA
   <213> Künstliche Sequenz
<220>
   <223> Beschreibung der künstlichen Sequenz:Oligonukleotide
<220>
   <221> misc_binding
   <222> (1)..(20)
<400> 4
   gcggggctcc atgggggtcg 20
<210> 5
   <211> 15
   <212> DNA
   <213> Künstliche Sequenz
<220>
   <223> Beschreibung der künstlichen Sequenz:Oligonukleotide
<220>
   <221> misc_binding
   <222> (1)..(15)
<400> 5
   cagctgcaac ccagc 15
<210> 6
   <211> 11
   <212> DNA
   <213> Künstliche Sequenz
<220>
   <223> Beschreibung der künstlichen Sequenz:Oligonukleotide
<220>
   <221> misc_binding
   <222> (1)..(11)
<400> 6
   tattccgtca t 11
<210> 7
   <211> 22
   <212> DNA
   <213> Künstliche Sequenz
<220>
   <223> Beschreibung der künstlichen Sequenz:Oligonukleotide
<220>
   <221> misc_binding
   <222> (1)..(22)
<400> 7
   ttccgtcatc gctcctcagg gg 22
<210> 8
   <211> 15
   <212> DNA
   <213> Künstliche Sequenz
<220>
   <223> Beschreibung der künstlichen Sequenz:Oligonukleotide
<220>
   <221> misc_binding
   <222> (1)..(15)
<400> 8
   cgctaccatg gtccc 15
<210> 9
   <211> 21
   <212> DNA
   <213> Kanstliche Sequenz
<220>
   <223> Beschreibung der künstlichen Sequenz:Oligonukleotide
<220>
   <221> misc_binding
   <222> (1)..(21)
<400> 9
   ggctgctgga gcggggcaca c 21
<210> 10
   <211> 15
   <212> DNA
   <213> Künstliche Sequenz
<220>
   <223> Beschreibung der künstlichen Sequenz:Oligonukleotide
<220>
   <221> misc_binding
   <222> (1)..(15)
<400> 10
   aacgttgagg ggcat 15
<210> 11
   <211> 18
   <212> DNA
   <213> Künstliche Sequenz
<220>
   <223> Beschreibung der künstlichen Sequenz:Oligonukleotide
<220>
   <221> misc_binding
   <222> (1) .. (18)
<400> 11
   gtgccggggt cttcgggc 18
<210> 12
   <211> 17
   <212> DNA
   <213> Künstliche Sequenz
<220>
   <223> Beschreibung der künstlichen Sequenz:Oligonukleotide
<220>
   <221> misc_binding
   <222> (1)..(17)
<400> 12
   cgagaacatc atcgtgg 17
<210> 13
   <211> 21
   <212> DNA
   <213> Künstliche Sequenz
<220>
   <223> Beschreibung der künstlichen Sequenz:Oligonukleotide
<220>
   <221> misc_binding
   <222> (1)..(21)
<400> 13
   ggagaacatc atggtcgaaa g 21
<210> 14
   <211> 22
   <212> DNA
   <213> Künstliche Sequenz
<220>
   <223> Beschreibung der künstlichen Sequenz:Oligonukleotide
<220>
   <221> misc_binding
   <222> (1)..(22)
<400> 14
   cccgagaaca tcatggtcga ag 22
<210> 15
   <211> 20
   <212> DNA
   <213> Künstliche Sequenz
<220>
   <223> Beschreibung der künstlichen Sequenz:Oligonukleotide
<220>
   <221> misc_binding
   <222> (1)..(20)
<400> 15
   ggggaaagcc cggcaagggg 20
<210> 16
   <211> 20
   <212> DNA
   <213> Künstliche Sequenz
<220>
   <223> Beschreibung der künstlichen Sequenz:Oligonukleotide
<220>
   <221> misc_binding
   <222> (1)..(20)
<400> 16
   cacccgcctt ggcctcccac 20
<210> 17
   <211> 18
   <212> DNA
   <213> Künstliche Sequenz
<220>
   <223> Beschreibung der künstlichen Sequenz:Oligonukleotide
<220>
   <221> misc_binding
   <222> (1)..(18)
<400> 17
   gggactccgg cgcagcgc 18
<210> 18
   <211> 20
   <212> DNA
   <213> Künstliche Sequenz
<220>
   <223> Beschreibung der künstlichen Sequenz:Oligonukleotide
<220>
   <221> misc_binding
   <222> (1)..(20)
<400> 18
   ggcaaacttt cttttcctcc 20
<210> 19
   <211> 19
   <212> DNA
   <213> Künstliche Sequenz
<220>
   <223> Beschreibung der künstlichen Sequenz:Oligonukleotide
<220>
   <221> misc_binding
   <222> (1)..(19)
<400> 19
   gggaaggagg aggatgagg 19
<210> 20
   <211> 21
   <212> DNA
   <213> Künstliche Sequenz
<220>
   <223> Beschreibung der künstlichen Sequenz:Oligonukleotide
<220>
   <221> misc_binding
   <222> (1) .. (21)
<400> 20
   ggcagtcatc cagcttcgga g 21
<210> 21
   <211> 18
   <212> DNA
   <213> Künstliche Sequenz
<220>
   <223> Beschreibung der künstlichen Sequenz:Oligonukleotide
<220>
   <221> misc_binding
   <222> (1)..(18)
<400> 21
   tctcccagcg tgcgccat 18
<210> 22
   <211> 19
   <212> DNA
   <213> Künstliche Sequenz
<220>
   <223> Beschreibung der künstlichen Sequenz:Oligonukleotide
<220>
   <221> misc_binding
   <222> (1)..(19)
<400> 22
   gcgctgatag acatccatg 19
<210> 23
   <211> 12
   <212> DNA
   <213> Künstliche Sequenz
<220>
   <223> Beschreibung der künstlichen Sequenz:Oligonukleotide
<220>
   <221> misc_binding
   <222> (1)..(12)
<400> 23
   ggaggcccga cc 12
<210> 24
   <211> 12
   <212> DNA
   <213> Künstliche Sequenz
<220>
   <223> Beschreibung der künstlichen Sequenz:Oligonukleotide
<220>
   <221> misc_binding
   <222> (1)..(12)
<400> 24
   ggtttcggag gc 12
<210> 25
   <211> 12
   <212> DNA
   <213> Künstliche Sequenz
<220>
   <223> Beschreibung der künstlichen Sequenz:Oligonukleotide
<220>
   <221> misc_binding
   <222> (1)..(12)
<400> 25
   tggtggaggt ag 12
<210> 26
   <211> 12
   <212> DNA
   <213> Künstliche Sequenz
<220>
   <223> Beschreibung der künstlichen Sequenz:Oligonukleotide
<220>
   <221> misc_binding
   <222> (1)..(12)
<400> 26
   gcatggtgga gg 12
<210> 27
   <211> 12
   <212> DNA
   <213> Künstliche Sequenz
<220>
   <223> Beschreibung der künstlichen Sequenz:Oligonukleotide
<220>
   <221> misc_binding
   <222> (1) . . (12)
<400> 27
   ttggcatggt gg 12
<210> 28
   <211> 12
   <212> DNA
   <213> Künstliche Sequenz
<220>
   <223> Beschreibung der künstlichen Sequenz:Oligonukleotide
<220>
   <221> misc_binding
   <222> (1) .. (12)
<400> 28
   gcctgggacc ac 12
<210> 29
   <211> 12
   <212> DNA
   <213> Kanstliche Sequenz
<220>
   <223> Beschreibung der künstlichen Sequenz:Oligonukleotide
<220>
   <221> nisc_binding
   <222> (1)..(12)
<400> 29
   cagcctggga cc 12
<210> 30
   <211> 12
   <212> DNA
   <213> Künstliche Sequenz
<220>
   <223> Beschreibung der künstlichen Sequenz:Oligonukleotide
<220>
   <221> misc_binding
   <222> (1)..(12)
<400> 30
   tgcagcctgg ga 12
<210> 31
   <211> 12
   <212> DNA
   <213> Künstliche Sequenz
<220>
   <223> Beschreibung der künstlichen Sequenz:Oligonukleotide
<220>
   <221> misc_binding
   <222> (1) .. (12)
<400> 31
   gtgcagcctg gg 12
<210> 32
   <211> 12
   <212> DNA
   <213> Künstliche Sequenz
<220>
   <223> Beschreibung der künstlichen Sequenz:Oligonukleotide
<220>
   <221> misc_binding
   <222> (1)..(12)
<400> 32
   ggtgcagcct gg 12
<210> 33
   <211> 12
   <212> DNA
   <213> Künstliche Sequenz
<220>
   <223> Beschreibung der künstlichen Sequenz:Oligonukleotide
<220>
   <221> misc_binding
   <222> (1)..(12)
<400> 33
   atgggtgcag cc 12
<210> 34
   <211> 12
   <212> DNA
   <213> Künstliche Sequenz
<220>
   <223> Beschreibung der künstlichen Sequenz:Oligonukleotide
<220>
   <221> misc_binding
   <222> (1)..(12)
<400> 34
   ggcttgaaga tg 12
<210> 35
   <211> 12
   <212> DNA
   <213> Künstliche Sequenz
<220>
   <223> Beschreibung der künstlichen Sequenz:Oligonukleotide
<220>
   <221> misc_binding
   <222> (1)..(12)
<400> 35
   gcagcccccg ca 12
<210> 36
   <211> 12
   <212> DNA
   <213> Künstliche Sequenz
<220>
   <223> Beschreibung der künstlichen Sequenz:Oligonukleotide
<220>
   <221> misc_binding
   <222> (1)..(12)
<400> 36
   gcagcagccc cc 12
<210> 37
   <211> 20
   <212> DNA
   <213> Künstliche Sequenz
<220>
   <223> Beschreibung der künstlichen Sequenz:Oligonukleotide
<220>
   <221> misc_binding
   <222> (1)..(20)
<400> 37
   tcccgcctgt gacatgcatt 20
<210> 38
   <211> 20
   <212> DNA
   <213> Künstliche Sequenz
<220>
   <223> Beschreibung der künstlichen Sequenz:Oligonukleotide
<220>
   <221> misc_binding
   <222> (1)..(20)
<400> 38
   gttctcgctg gtgagtttca 20
<210> 39
   <211> 18
   <212> DNA
   <213> Künstliche Sequenz
<220>
   <223> Beschreibung der künstlichen Sequenz:Oligonukleotide
<220>
   <221> misc_binding
   <222> (1)..(18)
<400> 39
   gcgtgcctcc tcactggc 18
<210> 40
   <211> 18
   <212> DNA
   <213> Künstliche Sequenz
<220>
   <223> Beschreibung der künstlichen Sequenz:Oligonukleotide
<220>
   <221> misc_binding
   <222> (1)..(18)
<400> 40
   gcagtaagca tccatatc 18
<210> 41
   <211> 20
   <212> DNA
   <213> Künstliche Sequenz
<220>
   <223> Beschreibung der künstlichen Sequenz:Oligonukleotide
<220>
   <221> misc_binding
   <222> (1)..(20)
<400> 41
   gcccaagctg gcatccgtca 20
<210> 42
   <211> 20
   <212> DNA
   <213> Künstliche Sequenz
<220>
   <223> Beschreibung der künstlichen Sequenz:Oligonukleotide
<220>
   <221> misc_binding
   <222> (1)..(20)
<400> 42
   cccccaccac ttcccctctc 20
<210> 43
   <211> 20
   <212> DNA
   <213> Künstliche Sequenz
<220>
   <223> Beschreibung der künstlichen Sequenz:Oligonukleotide
<220>
   <221> misc_binding
   <222> (1) .. (20)
<400> 43
   ctcccccacc acttcccctc 20
<210> 44
   <211> 19
   <212> DNA
   <213> Künstliche Sequenz
<220>
   <223> Beschreibung der künstlichen Sequenz:Oligonukleotide
<220>
   <221> misc_binding
   <222> (1)..(19)
<400> 44
   gctgggagcc atagcgagg 19
<210> 45
   <211> 21
   <212> DNA
   <213> Künstliche Sequenz
<220>
   <223> Beschreibung der künstlichen Sequenz:Oligonukleotide
<220>
   <221> misc_binding
   <222> (1)..(21)
<400> 45
   actgctgcct cttgtctcag g 21
<210> 46
   <211> 22
   <212> DNA
   <213> Künstliche Sequenz
<220>
   <223> Beschreibung der künstlichen Sequenz:Oligonukleotide
<220>
   <221> misc_binding
   <222> (1)..(22)
<400> 46
   caatcaatga cttcaagagt tc 22
<210> 47
   <211> 18
   <212> DNA
   <213> Künstliche Sequenz
<220>
   <223> Beschreibung der künstlichen Sequenz:Oligonukleotide
<220>
   <221> misc_binding
   <222> (1) . . (18)
<400> 47
   gcggcggaaa agccatcg 18
<210> 48
   <211> 18
   <212> DNA
   <213> Künstliche Sequenz
<220>
   <223> Beschreibung der künstlichen Sequenz:Oligonukleotide
<220>
   <221> misc_binding
   <222> (1)..(18)
<400> 48
   gtgtcggggt ctccgggc 18
<210> 49
   <211> 15
   <212> DNA
   <213> Künstliche Sequenz
<220>
   <223> Beschreibung der künstlichen Sequenz:Oligonukleotide
<220>
   <221> misc_binding
   <222> (1)..(15)
<400> 49
   cacgttgagg ggcat 15
<210> 50
   <211> 18
   <212> DNA
   <213> Künstliche Sequenz
<220>
   <223> Beschreibung der künstlichen Sequenz:Oligonukleotide
<220>
   <221> misc_binding
   <222> (1)..(18)
<400> 50
   gtcttccata gttactca 18
<210> 51
   <211> 18
   <212> DNA
   <213> Künstliche Sequenz
<220>
   <223> Beschreibung der künstlichen Sequenz:Oligonukleotide
<220>
   <221> misc_binding
   <222> (1)..(18)
<400> 51
   gatcaggcgt gcctcaaa 18
<210> 52
   <211> 21
   <212> DNA
   <213> Künstliche Sequenz
<220>
   <223> Beschreibung der künstlichen Sequenz:Oligonukleotide
<220>
   <221> misc_binding
   <222> (1)..(21)
<400> 52
   gatggagggc ggcatggcgg g 21
<210> 53
   <211> 11
   <212> DNA
   <213> Künstliche Sequenz
<220>
   <223> Beschreibung der künstlichen Sequenz:Oligonukleotide
<220>
   <221> misc_binding
   <222> (1)..(11)
<400> 53
   tattccgtca t 11

## Beschreibung

Die vorliegende Erfindung bezieht sich auf carboxyterminal und carboxy-/aminoterminal phosphorylierte Polyamidnukleinsäure-(PNA)-Derivate mit verbesserten Eigenschaften, deren Verwendung, sowie Mittel und Verfahren zu ihrer Herstellung.

Polyamidnukleinsäuren, auch Peptidnukleinsäuren (PNA) genannt, binden mit höherer Affinität als natürliche Oligonucleotide an komplementäre Zielsequenzen (DNA oder RNA) und haben gegenüber natürlicher DNA zudem den Vorteil, daß sie im Serum sehr stabil sind. PNA waren ursprünglich beschrieben als unnatürliche Nukleinsäure-Analoga, in denen das gesamte Zucker-Phosphat-Rückgrat durch N-(2-Aminoethyl)-glycin-Einheiten ersetzt ist (M. Egholm et al. (1991) Science 254, 1497-1500; WO 92/20702; M. Egholm et al. Nature (1993) 365, 566-568; P. Nielsen, (1994) Bioconjugate Chem. 5, 3-7; E. Uhlmann et al. (1998) Angewandte Chemie Int. Ed. Engl. 37, 2796-2823). Als Basen werden die in der Nucleotidchemie üblichen natürlich vorkommenden oder auch nicht natürlich vorkommenden Nucleobasen oder deren Prodrugformen benutzt, also Vorstufen, die erst im Organismus durch Biotransformation in die freie Base überführt werden. Darüberhinaus wurden PNAs beschrieben, in denen nicht alle Positionen des Rückgrates Basenreste tragen (Greiner et al. (1999) Helv. Chim. Acta 82, 2151), und in denen Aminoethylglycin durch komplexere Einheiten ersetzt ist (Uhlmann et al. (1998) Angewandte Chem. Int. Ed. 37, 2796; Falkiewicz (1999) Biochim. Pol., 46, 509-529).

Der ladungsneutrale Charakter des PNA-Rückgrats ist ein wichtiges Merkmal dieser Substanzklasse mit weitreichenden Konsequenzen. Es wird dem neutralen Charakter der PNA und der damit verbundenen verringerten Ladungsabstossung zugeschrieben, dass PNA selbst bei niedriger Salzkonzentration an komplementäre DNA und RNA bindet (z.B. Peptide Nucleic Acids: Protocols and Applications; Peter E. Nielsen und Michael Egholm (Edit.) Horizon Scientific Press, 1999, 3), wobei die Watson-Crick-Basenpaarungsregeln befolgt werden. Daher kann PNA im Prinzip für zahlreiche Anwendungen herangezogen werden, in denen sonst natürliche Oligonucleotide bzw. Oligonucleotid-Derivate eingesetzt werden. Darüber hinaus ergeben sich aber aufgrund der einzigartigen Bindungseigenschaften auch zahlreiche Anwendungen, die mit natürlichen Oligonucleotiden nicht möglich sind (siehe zum Beispiel: Peptide Nucleic Acids: Protocols and Applications; Peter E. Nielsen und Michael Egholm (Edit.) Horizon Scientific Press, 1999). Beispielsweise ist mit PNA eine Stranginvasion an doppelsträngiger DNA beobachtet worden.

Typische Beispiele zur Anwendung von PNA beinhalten deren Verwendung zur Inhibition der Genexpression durch sequenzspezifische Bindung an die zelluläre DNA oder RNA. "Antisense-Agenzien" sind kurze einzelsträngige Nukleinsäure-Derivate, die über Watson-Crick Basenpaarung an eine komplementäre mRNA binden, deren Übersetzung in das entsprechende Protein gehemmt werden soll (Uhlmann und Peyman (1990) Chem. Rev. 90, 543; Larsen et al. (1999) Biochem. Biophys. Acta 1489, 159). "Anti-Gen-Agenzien" binden über Hoogsteen-Basenpaarung in die große Furche der DNA-Doppelhelix unter Ausbildung einer Tripelhelix, wodurch die Transkription der Gene sequenzspezifisch gehemmt wird (Praseuth et al. (1999) Biochem. Biophys. Acta 1489, 181). Die Genexpression kann auch durch sogenannte "Decoy"-Oligomere, die die Bindungsregionen für Transkriptionsfaktoren nachahmen, spezifisch gehemmt werden. Durch die Behandlung mit Decoy-Agenzien lassen sich bestimmte Transkriptionsfaktoren sequenzspezifisch abfangen und dadurch eine Aktivierung der Transkription verhindern (Mischiati et al. (1999) J. Biol. Chem. 274, 33114). Eine weitere Gruppe von intrazellulär wirkenden Oligonucleotid-Derivaten, die Chimeraplasten, werden zur gezielten Genkorrektur herangezogen (Cole-Strauss et al. (1996) Science 273, 1386-1389).

PNA können daher als Arzneimittel und/oder Diagnostikum bzw. zur Herstellung von Arzneimitteln und/oder Diagnostika verwendet werden.

So offenbart die Schrift WO 00/33867A2 PNA Derivate zum Einsatz als Nukleinsäure Nachweissonden, die zur Herabsetzung unspezifischer Wechselwirkungen mit Nukleinsäuren derart modifiziert sind, dass sie im Inneren mindestens eine nichtnukleosidische Einheit mit einem PKa niedriger als 3,0 tragen. Der Nachteil dieser PNA Derivate besteht darin, dass durch die interne Modifikation eine negativ geladene Einheit in das Molekülinnere eingebaut wird, die eine Ladungsabstossung mit den Zielnukleinsäuren hervorruft; zudem kann die Modifikation im Inneren des PNA Derivats zu Störungen der Struktur der PNA-Nukleinsäure Duplexe führen.

Die Schrift EP 062 677 A2 offenbart PNA-DNA Oligomere zur Anwendung als Arzneimittel, als Gesonde oder Primer, wobei die Verknüpfung von PNA und DNA kovalent ist und mindestens einen organischen Rest oder eine Bindung mit mindestens einem Atom aus der Reihe C, N, O oder S beinhaltet.

Aus der US Patentschrift 5,874,553 sind oligomere Phosphonsäuremonoester (PMENAs) sowie Phosphonsäurederivate bekannt, welche auf der Verbindung von PMENAs mit PNAs beruhen und als Inhibitoren der Genexpression, Nachweissonden und dergleichen dienen. Die Phosphonatrest befinden sich in den hier offenbarten Molekülen ebenfalls ausschließlich im Inneren der Moleküle und dienen als Brücken zur kovalenten Verbindung der monomeren Einheiten innerhalb der PMENAs oder zwischen PMENAs und PNAs.

Für diagnostische Zwecke und in der Molekularbiologie kann PNA beipielsweise nach Markierung mit Biotin, Fluorescein oder anderen Labeln als spezifische Hybridisierungssonde eingesetzt werden. Für die Einführung der Markergruppen sind in der Literatur bislang vier Methoden beschrieben (Oerum et al. (1999), in Peptide Nucleic Acids: Protocols and Applications, Seiten 81-86; Lohse et al. (1997) Bioconjugate Chem. 8, 503). Die erste Methode beruht auf der Markierung der freien (entschützten) PNA nach deren Synthese in Lösung. Dabei wird der Aminoterminus der PNA mit einer aktivierten Carbonsäure oder einem Isothiocyanat umgesetzt. Oft werden aber zusätzliche Lysin-Reste in die PNA eingeführt, die dann mit Fluoresceinisothiocyanat (FITC) umgesetzt werden.

Bei der zweiten Methode wird die geschützte PNA noch an der Festphase am Aminoterminus mit aktivierten Carbonsäure-Derivaten oder Isothicyanaten modifiziert. Diese Methode eignet sich nur für Markergruppen, die unter den Bedingungen der Entschützung der PNA und während der Abspaltung vom Träger stabil sind. Als reaktive Reagenzien werden in beiden Fällen bevorzugt Isothiocyanate (P. Wittung et al., (1995) FEBS Lett. 375, 27) oder aktivierte Carbonsäuren, wie beispielsweise N-Hydroxysuccinimidester (NHS), eingesetzt (Oerum et al., 1999). Ein Nachteil der Reaktion mit den NHS-Derivaten ist, dass sie oft nur in schlechten Ausbeuten gelingt. Daher wird häufig 8-Amino-3,6-dioxaoctansäure als Linker bzw. Spacer zwischen PNA und Markergruppe kondensiert (Oerum et al., 1999). Beide Verknüpfungen erfolgen über Amidbindungen bzw. Thioharnstoffbindungen, die als solche aber eher zu Unlöslichkeit führen. Alternativ werden die Carbonsäuren mit Hilfe von in der Peptid-Chemie üblichen Aktivatoren, wie beispielsweise HBTU, TBTU oder HATU zur Reaktion gebracht.

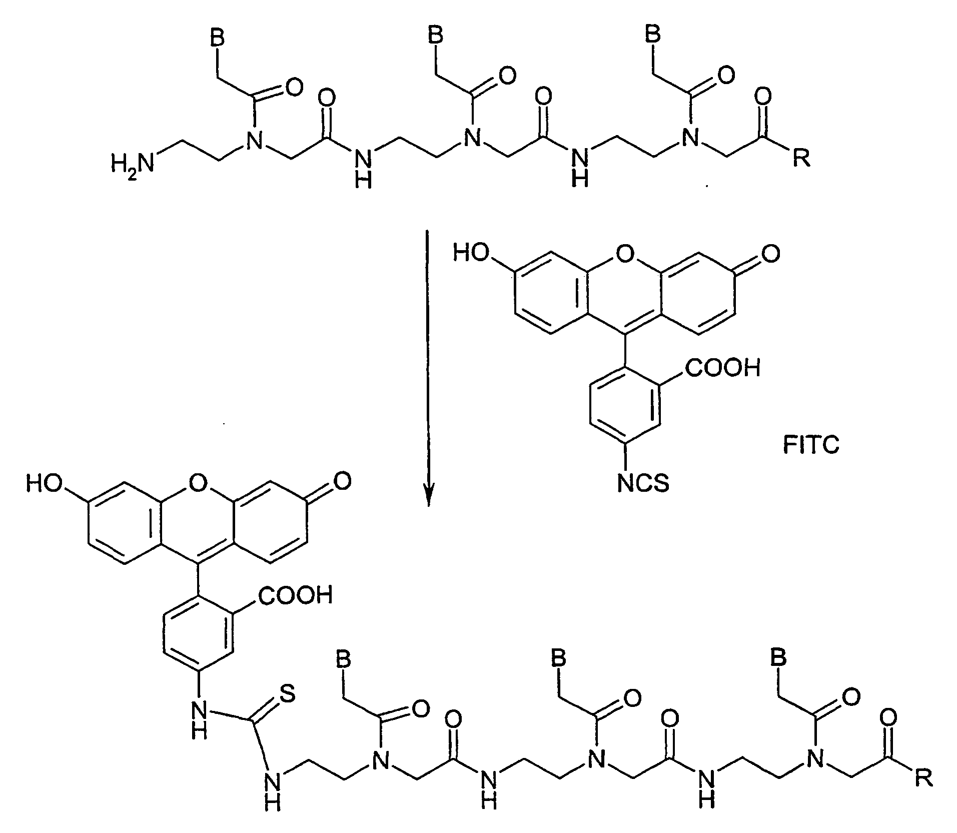

Bei einer dritten Methode werden Fluorescein-konjugierte Monomere bei der Festphasensynthese von PNA verwendet, wobei die Fluoreszenz-Markierung über eine Amidbindung erfolgt (Lohse et al. (1997) Bioconjugate Chem. 8, 503), die wiederum zu relativ schwerlöslichen Konjugaten führt.

Eine vierte Methode verwendet PNA-Peptid-Konjugate, in denen der Peptid-Teil ein Substrat für eine Protein-Kinase bildet (Koch et al. (1995) Tetrahedron Lett. 36, 6933). Auf diese Weise wird also nicht der PNA-Teil modifiziert, sondern der Serin-Rest des Peptid-Segments wird enzymatisch phosphoryliert. Mit dieser Methode kann also nur radioaktives Phosphat, aber beispielsweise kein Fluorescein oder Biotin in das Peptid-Segment des PNA-Peptid-Konjugats eingeführt werden.

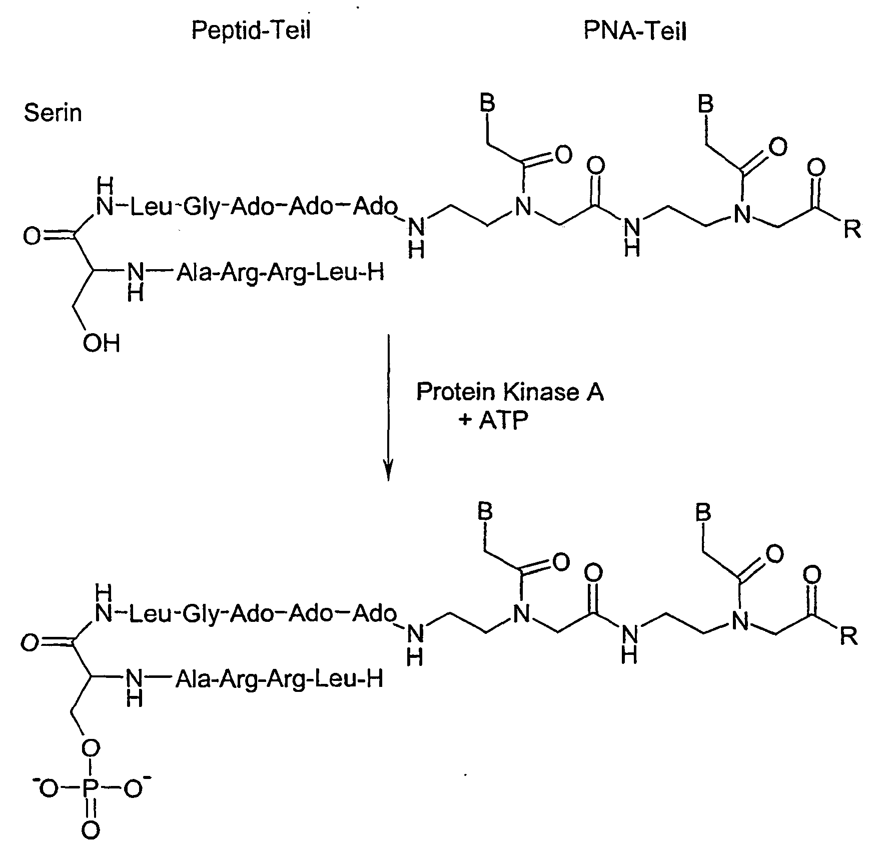

Es ist bekannt, dass PNA in wässriger Lösung, also auch unter physiologischen Bedingungen, zur Aggregation neigt. PNA ist daher in wässrigem Puffer schlecht löslich und steht dann nicht für die Hybridisierung an komplementäre Sequenzen zur Verfügung. PNA zeigt zudem eine hohe Affinität zu verschiedenen Materialien wie Sephadex® (Fa. Pharmacia), Bond Elut® (Fa. Varian), oder verschiedene HPLC-Chromatographiematerialien, die bei der Aufreinigung der Oligomeren Verwendung finden, so daß die PNA oft nur in schlechten Ausbeuten zu isolieren ist. Daher ist es notwendig, die PNA mit Lysin oder anderen positiv geladenen Aminosäuren (über den C-Terminus) zu konjugieren (Egholm et al (1992) J. Am. Chem. Soc. 114, 1895). Guaninreiche PNA-Sequenzen tendieren ganz besonders zur Aggregation, weshalb von der Verwendung solcher PNA abgeraten wird (siehe "Guidelines for sequence design of PNA oligomers" in Peptide Nucleic Acids: Protocols and Applications (1999) Seiten 253-255). Besonders schwerlöslich sind beispielsweise längere Fluorescein-markierte PNA-Oligomere, wobei die Zugabe eines organischen Lösemittels und Erwärmen auf 50°C empfohlen wird.

Die Reinigung der schwerlöslichen lipophilen PNA-Derivate ist besonders schwierig. Bei der HPLC werden oft mehrere Peaks detektiert, die auf PNA-Aggregate zurückzuführen sind. Die für die Reinigung und Trennung von Nukleinsäuren häufig angewandte Technik der Polyacrylamid (PAA)-Gelelektrophorese ist für diese PNA-Derivate nicht möglich.

Bei den oben beschriebenen Methoden der Derivatisierung von PNA wird die Marker-Gruppe stets durch Knüpfung einer Amidbindung oder Thioamidbindung eingeführt, wobei relativ schwerlösliche PNA-Derivate gebildet werden. Insbesondere bei der Einführung lipophiler Reste, wie beispielsweise des Fluoresceins, entstehen schwerlösliche PNA-Derivate. Die Einführung von Markern an beiden Enden der PNA ist technisch noch schwieriger und führt im allgemeinen zu einer noch schlechteren Löslichkeit. Ausserdem sind keine effiziente Methoden für eine gleichzeitige amino- und carboxyterminale Derivatisierung von PNA, insbesondere durch Festphasensynthese beschrieben. Da die Markierungs-Reaktionen zudem oft in schlechten Ausbeuten verlaufen, besteht eine zu lösende Aufgabe darin, neue PNA-Derivate bereitzustellen, welche in hohen Ausbeuten herzustellen sein sollen und vorteilhaften Eigenschaften aufweisen, wie verbesserter Löslichkeit, verbessertem Bindungsverhalten, besserer zellulärer Aufnahme aufweisen sollen, und die zudem den Einsatz effektiver Reinigungsmethoden für die PNA-Oligomeren erlaubt.

Die Aufgabe wird erfindungsgemäss gelöst durch die Bereitstellung von PNA-Derivaten der Formel I 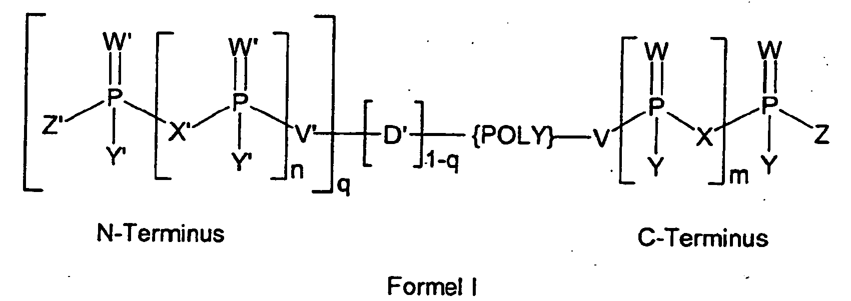 wobei
- q: gleich 0 oder 1 ist,
- D': gleich Hydroxy, Mercapto, Amino, Alkylamino oder Acylamino, vorzugsweise Acetylamino, ist,
- V: unabhängig voneinander gleich Sauerstoff, Schwefel, NR₁ ist,
- V': unabhängig voneinander gleich Sauerstoff, Schwefel, NR₁, eine Gruppe U-(CR₃R₄)_{u'}-C(O)-NH oder eine Gruppe U-(CH₂CH₂O)_{u'}-CH₂-C(O)-NH ist,
- U: unabhängig voneinander gleich Sauerstoff, Schwefel oder NH ist,
- u': unabhängig voneinander gleich 1 bis 10 ist, vorzugsweise 1 bis 4, besonders bevorzugt 1,
- W und W': unabhängig voneinander gleich Sauerstoff, Schwefel oder NR₁ sind,
- Y und Y': unabhängig voneinander gleich Hydroxy, Mercapto, Oxyanion, Thioat oder NR₁ R₂ sind,
- X und X': unabhängig voneinander gleich eine Gruppe U-(C₂-C₂₂₋Alkandiyl)-U oder eine Gruppe U-(CH₂CH₂-O)_{u'} ist, oder gleich eine Markergruppe, oder eine Gruppe zur Quervemetzung, oder eine Gruppe, die die intrazelluläre Aufnahme begünstigt, oder eine Gruppe, die die Bindungsaffinität des PNA-Derivats an Nukleinsäuren steigert, ist, beispielsweise ein bifunktioneller Fluorescein-, Rhodamin-, TAMRA-, Biotin-, Pyren-, Dinitrophenyl-, Cholesteryl-, Acridin-, Adamantyl-, Vitamin E-, Cyaninfarbstoff-, Dabcyl-, Edans-, Lexitropsin-, Psoralen-, BODIPY-, ROX-, R6G- oder Digoxigenin-Rest,
- Z und Z': unabhängig voneinander gleich Hydroxy, Mercapto, Oxyanion, Thioat oder NR₁ R₂, C₁-C₂₂-Alkyl, C₁-C₈-Arylalkyl, C₁-C₂₂-Alkyl-U, C₁-C₈-Arylalkyl-U, Hydroxy-C₁-C₁₈-U, Aminoalkyl-U, Mercaptoalkyl-U ist,
oder eine Gruppe der Formel R₇(CH₂CH₂-O)ₘ ist, wobei R₇ gleich Hydroxy, Amino oder C₁-C₂₂-Alkoxy und m gleich 1 bis 100 ist, vorzugsweise 2 bis 10,
oder gleich eine Markergruppe, oder eine Gruppe zur Quervemetzung, oder eine Gruppe, die die intrazelluläre Aufnahme begünstigt, oder eine Gruppe, die die Bindungsaffinität des PNA-Derivats an Nukleinsäuren steigert, ist,
beispielsweise ein monofunktioneller oder bifunktioneller Fluorescein-, Rhodamin-, TAMRA-, Biotin-, Pyren-, Dinitrophenyl-, Cholesteryl-, Acridin-, Adamantyl-, Vitamin E-, Cyaninfarbstoff-, Dabcyl-, Edans-, Lexitropsin-, Psoralen-, BODIPY-, ROX-, R6G-oder Digoxigenin-Rest,
- R₁ und R₂: unabhängig voneinander ein Rest bestehend aus Wasserstoff oder C₁-C₆-Alkyl bedeuten, vorzugsweise Wasserstoff,
- R₃ und R₄: unabhängig voneinander ein Rest bestehend aus Wasserstoff oder C₁-C₆-Alkyl oder den Rest einer Aminosäure-Seitenkette bedeuten, vorzugsweise Wasserstoff, wobei benachbarte Reste R₃ und R₄ in V' auch einen C₅-C₈-Cycloalkyl-Ring bilden können,
- n: gleich 0 bis 10 ist, vorzugsweise 0 bis 3,
- m: gleich 0 bis 10 ist, vorzugsweise 0 bis 3,
mit der Massgabe, dass mindestens ein Rest Y, Y', Z oder Z' gleich Hydroxy, Mercapto, Oxyanion oder Thioat ist.

Die Gruppe {POLY} wird beschrieben durch die Formel II, 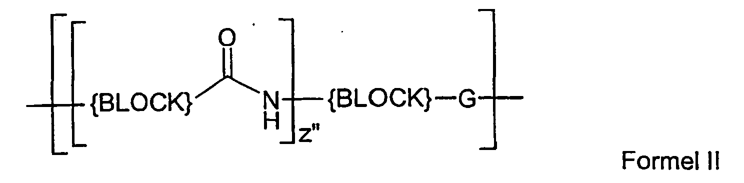 wobei {BLOCK} unabhängig voneinander eine Gruppe ist ausgewählt aus Formel IIIA, 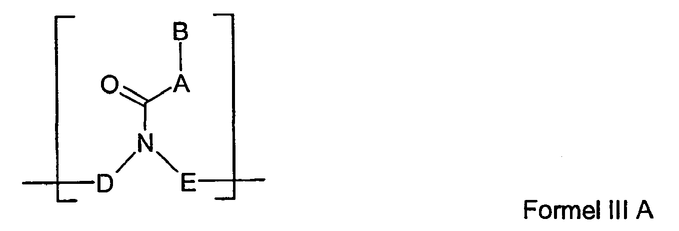 oder aus Formel IIIB (Greiner et al. (1999) Helv. Chim Acta 82, 2151), 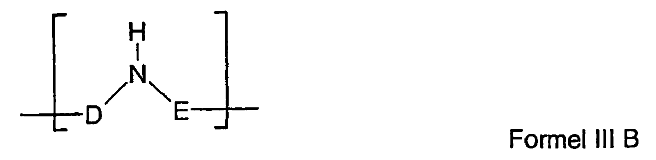 oder aus den Formeln IV A bis IV G (Uhlmann et al. (1998) Angewandte Chem. Int. Ed. 37, 2796; Falkiewicz (1999) Biochim. Pol., 46, 509-529), 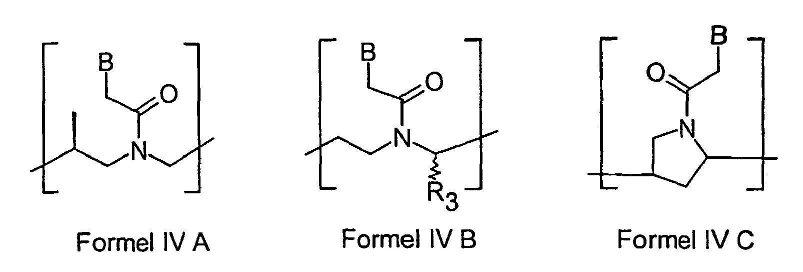 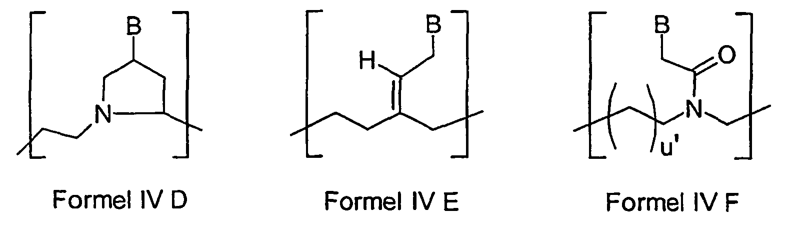 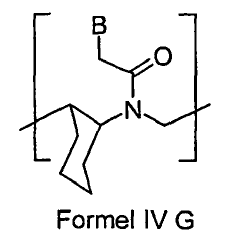 wobei jeder Baustein {BLOCK} verschieden sein kann,
und wobei ferner gilt, dass
- z": gleich 0 bis 100 ist, vorzugsweise 1-20, besonders bevorzugt 4-15,
- G: ausgewählt wird aus den Gruppen (CR₅R₆)_{u'}, C(O)NH-(CR₁R₂)_{t'} oder C(O)NH-(CH₂CH₂O)_{u'}-CH₂CH₂ ist, wobei u' die obige Bedeutung hat und t' gleich 2 bis 10 ist, vorzugsweise 6,
- A: unabhängig voneinander eine Gruppe (CR₁R₂)ₛ ist, wobei s gleich 1 bis 3 ist, vorzugsweise 1,
- B: unabhängig voneinander entweder ein aromatischer Rest, der auch heteroaromatischen Charakter besitzen kann, oder Wasserstoff, oder Hydroxy oder C₁-C₁₈-Alkyl ist,
oder eine in der Nucleotidchemie übliche natürlich vorkommende oder eine nicht natürlich vorkommende Nucleobase oder deren Prodrugform ist,
mit der Maßgabe, dass mindestens ein Rest B eine Nucleobase ist,
- D: unabhängig voneinander eine Gruppe (CR₃R₄)ₜ ist, wobei t gleich 2 bis 10 ist, vorzugsweise 2 bis 4, besonders bevorzugt 2,
- E: unabhängig voneinander eine Gruppe (CR₅R₆)_{u'}- ist, wobei benachbarte Reste R₅ und R₆ auch einen C₅-C₈-Cycloalkyl-Ring oder eine Spiroverbindung bilden können,
- R₅ und R₆: unabhängig voneinander einen Rest bestehend aus Wasserstoff oder-C₁-C₆-Alkyl oder den Rest einer Aminosäure-Seitenkette bedeuten, vorzugsweise Wasserstoff,
und wobei u', R₁, R₂, R₃ und R₄ die gleiche Bedeutung wie oben beschrieben haben,
sowie physiologisch verträglichen Salze der PNA-Derivate der Formel I. Physiologisch verträglichen Salze sind u.a. beschrieben in Remingtons Pharmaceutical Science (1985) Mack Publishing Company, Easton, PA, USA, Seite 1418. Bevorzugt sind Ammoniumsalze, Trialkylammoniumsalze, Alkalimetallsalze (wie Natrium- und Kaliumsalze) und Erdalkalisalze (wie Magnesium- und Calciumsalze). Besonders bevorzugt sind Natriumsalze.

Unter Markergruppen (Labeln) versteht man dabei Gruppen, die eine qualitative oder quantitative Bewertung der chemischen oder biologischen Aktivität der PNA-Derivate erlauben, beispielsweise Biotin oder Fluoreszein. Unter Quervernetzung (Cross-Linking) versteht man die Ausbildung intra- bzw. intermolekularer Bindungen zwischen räumlich benachbarten Funktionalitäten Eine Gruppe zur Quervernetzung ist beispielsweise die Psoralengruppe.

Als überraschender positiver Effekt zeigte sich, dass die Einführung eines endstaandiger Phosphoryl-Restes beispielsweise als Phosphat oder auch in Form einer lipophilen Derivatisierung (z. B. als Hexadecylphosphodiester) die Affinität der PNA an komplementäre DNA oder RNA erhöht. Dieser Effekt war nicht zu erwarten, da die starke Binding von PNA an komplementäre DNA oder RNA dem neutralen Charakter der PNA und der damit verbundenen reduzierten Ladungsabstossung zugeschrieben wurde (z.B. Peptide Nucleic Acids: Protocols and Applications; Peter E. Nielsen und Michael Egholm (Edit.) Horizon Scientific Press, 1999, 3).

Besonders effektiv erfolgte die Einführung des Biotins über einen Phosphorylrest. Die biotinylierte PNA der Formel I (X, X', Z und/oder Z' = Biotinrest) zeigten als Hybridisierungssonden bessere Bindungseigeilschaften und weniger störende unspezifische Hintergrund-Effekte als entsprechende biotinylierte DNA-Sonden.

Im Gegensatz zur ungeladenen PNA können die erfindungsgemäßen PNA-Derivate der Formel I auch im elektrischen Feld wandern, wodurch eine Mikrolokalisierung und eine Konzentrierung an immobilisierten komplementären Nukleinsäurederivaten möglich ist. Für die polyanionischen Oligonucleotide wurde diese Methode zur raschen Bestimmung von Basenmisspaarungen mit Hilfe des elektrischen Felds bereits beschrieben (Sosnowski et al. (1997) Proc. Natl. Acad. Sci. U.S.A. 94, 1119).

Die Hydroxy- bzw. Mercapto-Substituenten der Phosphoryl-Reste der erfindungsgemässen PNA-Derivate können in einem pH-Bereich von 4,5 bis 14, vorzugsweise 6,5 bis 12, besonders bevorzugt 6,5 bis 9 deprotoniert werden. Die Eigenschaft der lonisierbarkeit der Phosphoryl-Reste kann vorteilhaft zur Reinigung der Verbindungen der Formel I ausgenutzt werden. Einerseits können die Verbindungen der Formel I durch Elektrophorese, beispielsweise Polyacrylamid-Gelelektrophorese, gereinigt werden. Andererseits ist eine Reinigung mit Hilfe von Anionenaustauschern möglich. Dabei werden die gewünschten Produkte bevorzugt durch Anwendung eines Salzgradienten, beispielsweise eines Kochsalz-Gradienten, oder eines pH-Gradienten eluiert.

Besonders einfach und effizient lassen sich die erfindungsgemäßen PNA-Derivate der Formel I über Anionenaustauscher reinigen. Es zeigte sich, dass die ungeladenen Nebenprodukte nicht auf dem Anionenaustauscher retardiert werden, während das geladene Produkt auf der Säule haftete. Nach Waschen mit Wasser konnte das gewünschte Produkt mit Essigsäure oder einer Kochsalzlösung in reiner Form isoliert werden. Als Anionenaustauscher verwendet man bevorzugt starke Anionenaustauscher, oder Mixed-Mode-Phasen, wie beispielsweise ®Oasis MAX (Waters GmbH, Eschbom).

Weiterhin zeigte sich, dass die erfindungsgemäßen Verbindungen der Formel I generell besser in wässrigem Medium löslich sind als die entsprechenden PNA-Oligomeren ohne den Phosphoryl-Rest. Dies macht sich ganz besonders bei den lipophilen Derivaten, wie beispielsweise den Fluorescein-Derivaten oder Hexadecyl-Derivaten, in Form einer stark verbesserten Löslichkeit in wässrigem Medium bemerkbar.

Die Erfindung betrifft besonders PNA-Derivate, in denen A und E gleich CH₂ sind. Weiterhin betrifft die Erfindung besonders PNA-Derivate, in denen D gleich (CH₂)₂ sind. Bevorzugt sind ferner PNA-Derivate der Formel I, in denen W und W' gleich Oxo sind, weiterhin solche, in denen Y und Y' gleich Hydroxy oder Oxyanion sind, und solche in denen V und V' gleich Oxy sind.

Beispiele für natürliche Basen sind Adenin, Cytosin, 5-Methylcytosin, Guanin, Thymin und Uracil. Beispiele für unnatürliche Basen sind Purin, 2,6-Diaminopurin, N⁴N⁴-Ethanocytosin, N⁶N⁶-Ethano-2,6-diaminopurin, 5-(C₃-C₆)-Alkinyl-uracil, 5-(C₃-C₆)-Alkinyl-cytosin, 5-(1-Propargylamino)-uracil, 5-(1-Propargylamino)-cytosin, Phenoxazin, 9-Aminoethoxyphenoxazin, 5-Fluor-uracil oder Pseudoisocytosin, 5-(Hydroxymethyl)uracil, 5-Aminouracil, Pseudouracil, Dihydrouracil, 5-(C₁-C₆)-Alkyl-uracil, 5-(C₁-C₆)-Alkyl-cytosin, 5-(C₂-C₆)-Alkenylcytosin, 5-Fluorcytosin, 5-Chloruracil, 5-Chlorcytosin, 5-Bromuracil, 5-Bromcytosin, 7-Deazaadenin, 7-Deazaguanin, 8-Azapurin, oder 7-Deaza-7-substituierte Purine.

Für PNA-Derivate, die nur C-terminal einen Phosphoryl-Rest tragen (für die q gleich 0 ist), kann der N-Terminus mit einer Peptidsequenz verknüpft sein. Als Peptidsequenzen kommen bevorzugt solche in Betracht, die Organverteilung oder die zelluläre Lokalisierung des PNA optimieren, wie beispielsweise Transportan, Insulin-like Growth Factor, Nuclear Localisation Signale oder andere Carriersequenzen (Larsen et al. (1999) Biochim. Biophys. Acta 159-166). Das Peptid kann auch als Affinitäts-Tag dienen, wie etwa eine (His)₆ Kette.

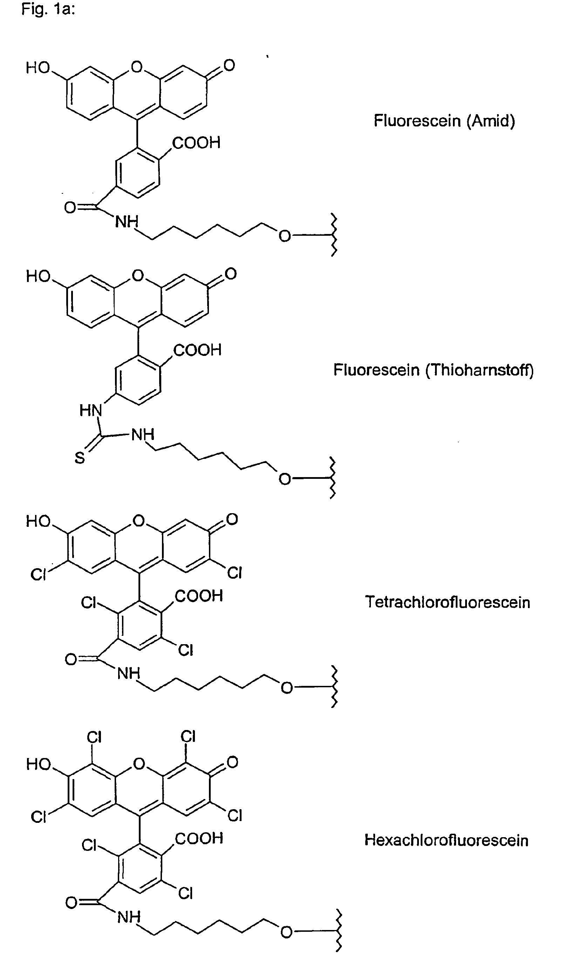

Die vorliegende Erfindung ermöglicht eine breite Variation der Reste X, X', Z und Z' (Beispiele sind in Figuren 1a, 1b, 2a, 2b, 3a und 3b gegeben) und erlaubt so die Einführung unterschiedlicher spezifischer Funktionsmerkmale in PNA.

Eine bevorzugte Ausführungsform von Z bzw. Z' ist ein C₁- bis C₂₂-Alkyl-Rest. Bevorzugt sind auch C₁- bis C₂₂-Alkoxy-Reste, insbesondere C₁₆-AlkoxyReste. Andere bevorzugte Reste sind Hydroxy-(C₁-C₁₈-Alkoxy)-Reste, insbesondere HO(CH₂)₃₋₁₂O. Weiterhin bevorzugt sind Aminoalkoxy-Reste, insbesondere 6-Aminohexoxy- und 5-Aminopentoxy-Reste. Weiterhin bevorzugt sind Reste der Formel R₇-(CH₂CH₂-O)ₘ, wobei R₇ gleich Hydroxy, Amino oder C₁-C₂₂-Alkoxy ist, bevorzugt aber Hydroxy ist, und m gleich 0 bis 100, bevorzugt 2 bis 10 ist. Besonders bevorzugt sind HO(CH₂CH₂-O)₂, HO(CH₂CH₂-O)₆ und H₂N-(CH₂CH₂-O)₂. Weitere bevorzugte Beispiele von Z bzw. Z' umfassen Mercaptoalkoxy-Reste, insbesondere 6-Mercaptohexyloxy.

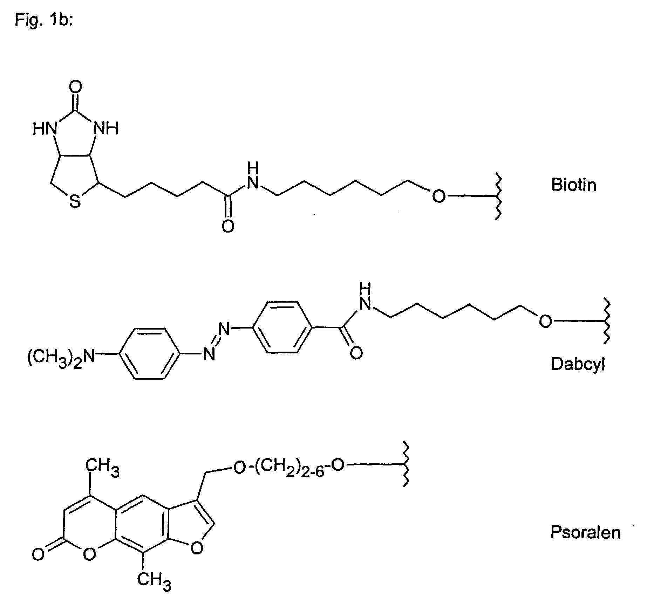

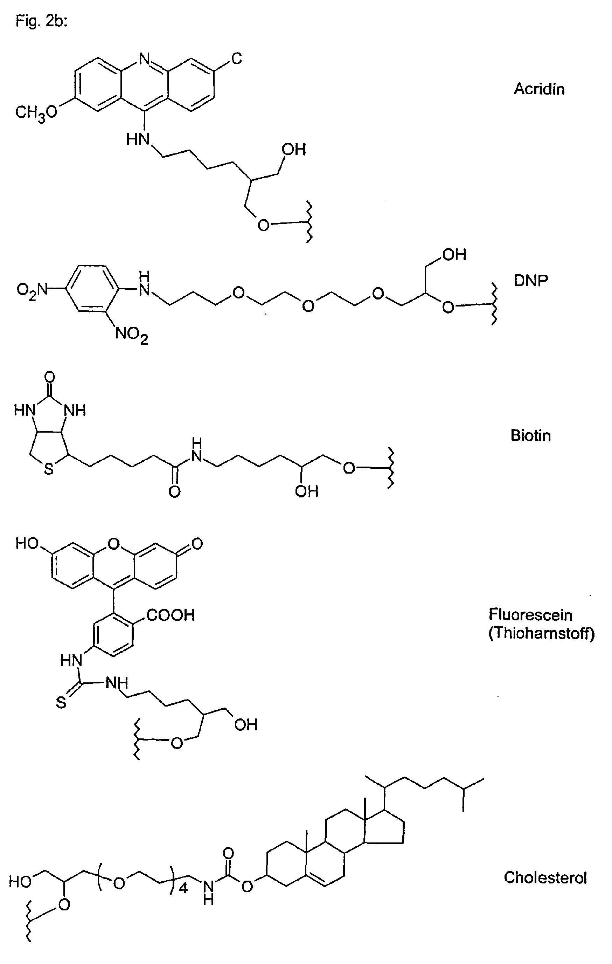

In einer weiteren bevorzugten Ausführungsform umfasst Z bzw. Z' eine fluoreszierende Gruppe wie Fluorescein, Rhodamin, TAMRA oder einen Cyanin-Farbstoff. Bevorzugte fluoreszierende Gruppen sind in den Figuren 1a bis 3b zu finden. Ganz besonders bevorzugt ist auch Biotin für Z. Andere bevorzugte Gruppen für Z umfassen Dabcyl, Psoralen, Acridin, DNP und Cholesterol (Figuren 1b und 2b), BODIPY-, ROX- oder R6G-Reste (Su-Chun Hung et al. (1998) Analytical Biochemistry 255, 32-38) und Digoxigenin (Tarrason et al., Methods in Enzyology (1999) Vol. 313, 257-268).

Darüberhinaus kann Z bzw. Z' gleich einer Gruppe bestehend aus einem monofunktionellen oder einem bifunktionellen Fluorescein-, Rhodamin-, TAMRA-, Biotin-, Pyren-, Dinitrophenyl-, Cholesteryl-, Acridin-, Adamantyl-, Vitamin-E-, Cyaninfarbstoff-, Dabcyl-, Edans-, Lexitropsin-, oder Psoralen-Rest sein. Monofunktionelle Endgruppen sind beispielhaft in den Figuren 1a, 1 b, 2a und 3a ausgeführt, bifunktionelle verbrückende Gruppen sind in Figuren 2b und 3b aufgeführt. In einer anderen bevorzugten Ausführungsform ist n und/oder m unabhängig voneinander gleich 0, d.h. der PNA-Teil trägt am N- und/oder am C-Terminus jeweils nur einen Phosphoryl-Rest.

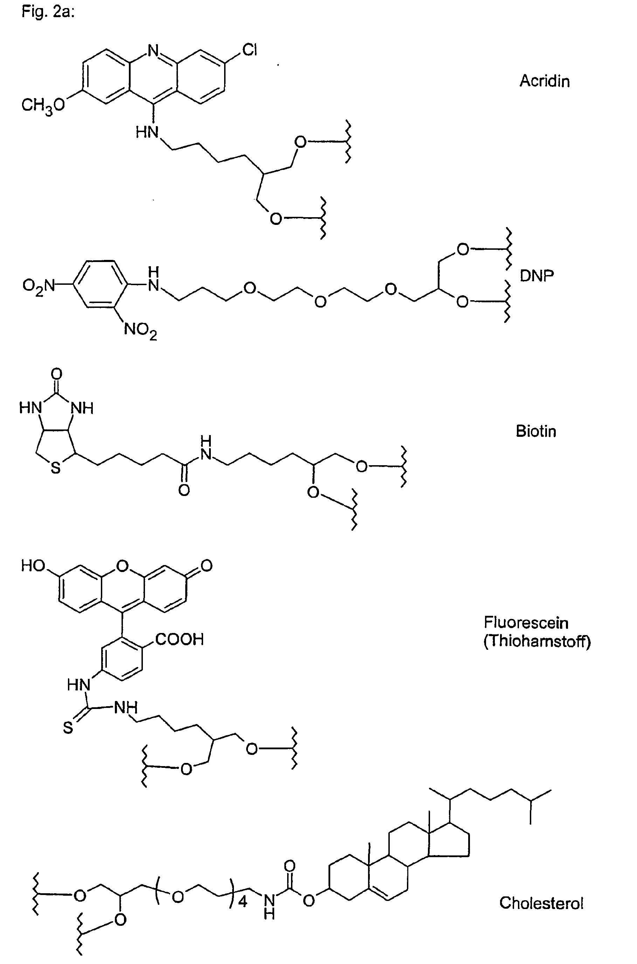

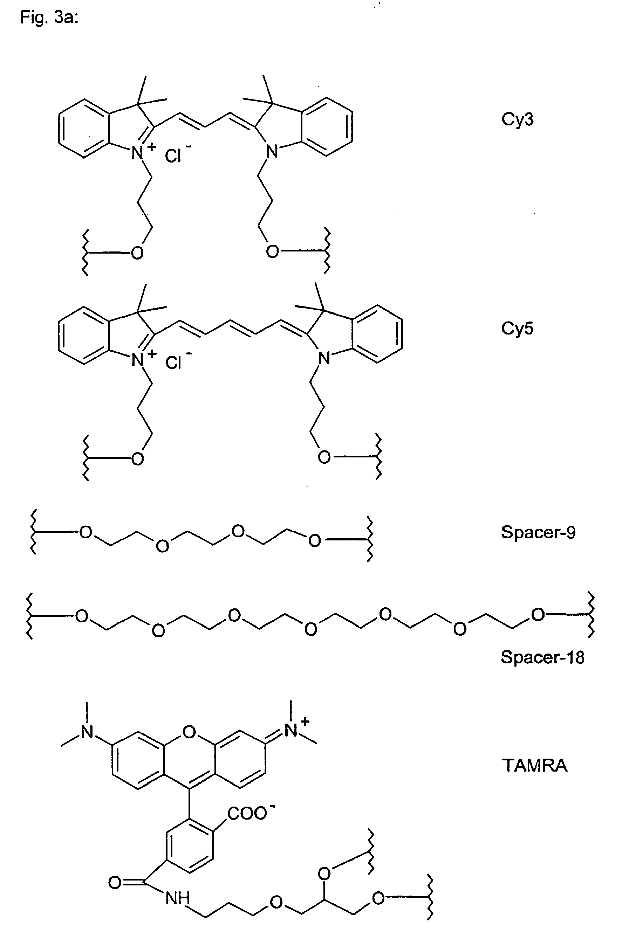

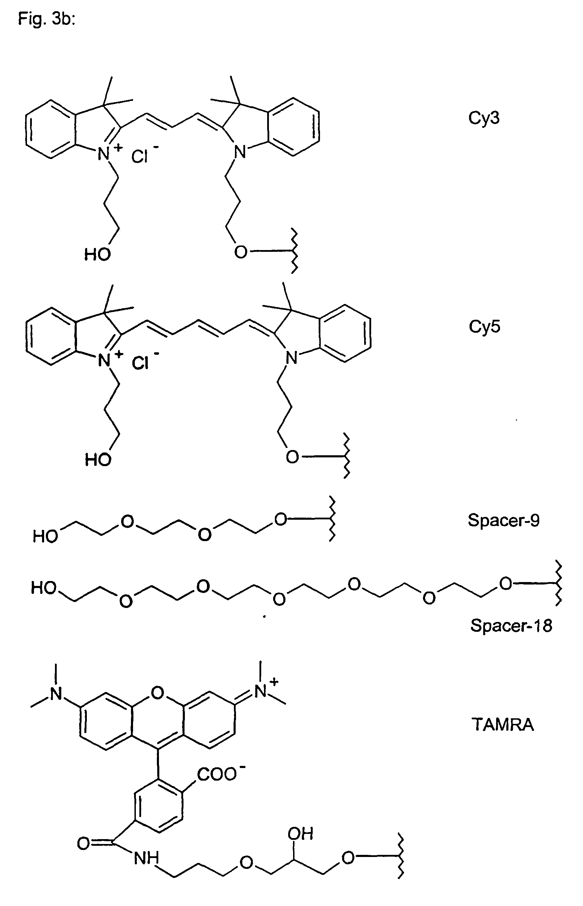

Eine bevorzugte Ausführungsform von X bzw. X' ist U-(C₂-C₂₂-Alkandiyl)-U, insbesondere O-(C₂-C₂₂-Alkandiyl)-O, besonders bevorzugt O-(CH₂)₂₋₆-O. Eine andere bevorzugte Ausführungsform von X bzw. X' ist eine Gruppe der Formel U-(CH₂CH₂-O)ᵤ, ist, wobei u' gleich 1 bis 10 ist, bevorzugt 1 bis 6, und wobei U bevorzugt Oxy ist. In einer weiteren bevorzugten Ausführungsform umfasst X bzw. X' eine fluoreszierende Gruppe wie Fluorescein, Rhodamin, TAMRA oder einen Cyanin-Farbstoff, beispielsweise Cy3® (Fa. Amersham Pharmacia Biotech). Bevorzugte bifunktionelle Gruppen sind in den Figuren 2a und 3a zu finden. Ganz besonders bevorzugt ist auch Biotin für X bzw. X'. Andere bevorzugte Gruppen sind Dabcyl-, Psoralen-, Acridin-, DNP-, Cholesterol-, BODIPY-, Lexitropsin-, Digoxigenin-, ROX- und R6G-Reste.

Die unterschiedlichen Reste für X, X', Z und Z' in Formel I können unterschiedliche Funktionen erfüllen. Die Fluorescein-Reste haben weitreichende Anwendungen bei der DNA-Sequenzierung, Signalamplifikation oder als Marker zur Bestimmung der zellulären Aufnahme von PNA. Die Cyanin-Farbstoff-Reste (Cy3® und Cy5®) ergeben ein wesentlich intensiveres und länger anhaltendes Fluoreszenzsignal als Fluorescein selbst. Der Psoralen-Rest wird zur Quervernetzung (Cross-Linking) mit komplementären Nukleinsäuren eingesetzt. Der Acridin-Rest ist ein effektiver Interkalator und kann damit die Bindungsaffinität der PNA verstärken. Biotin-, Acridin- und Psoralen-Derivate können auch für Antisense-Experimente eingesetzt werden. Weiterhin können Hexadecyloxy- und Cholesterol-Derivate zur Erhöhung der Membrangängigkeit der PNA angewandt werden. DNP-markierte Verbindungen der Formel I lassen sich mit anti-DNP-Antikörpem nachweisen. Aminoalkoxy-Reste lassen sich zur Kupplung weiterer Gruppen, wie beispielsweise Lexitropsin (vgl. Beispiel 17; PNA-16), nutzen. In ähnlicher Weise lassen sich auch Mercaptoalkoxy-Gruppen zur weiteren Derivatisierung nutzen.

Die Erfindung betrifft weiterhin die Verwendung der PNA-Derivate der Formel I als Arzneimittel. Diese Arzneimittel können zur Prävention und/oder Behandlung von Krankheiten, die mit der Expression bzw. einer Überexpression bestimmter Gene einhergehen, eingesetzt werden. Weiterhin betrifft die Erfindung die Verwendung von PNA-Derivaten als Diagnostikum. Diese Diagnostika können zur Früherkennung der oben genannten Krankheiten eingesetzt werden.
Als Arzneimittel bzw. Diagnostikum lassen sich die PNA-Derivate der Formel in Abhängigkeit ihrer Sequenz als Antisense-, Antigene-, Decoy-, und Chimeraplast-Agenzien nutzen.

Die erfindungsgemäßen PNA-Derivate werden insbesondere zur Herstellung von Arzneimitteln zur Behandlung von Krankheiten verwendet, bei denen definierte Gene durch Überexpression ursächlich bzw. beteiligt ist.

Diese Arzneimittel können beispielsweise zur Behandlung von Erkrankungen verwendet werden, die durch Viren hervorgerufen werden, beispielsweise durch CMV, HIV, HSV-1, HSV-2, Influenza, VSV, Hepatitis B oder Papilloma Viren, wobei die entsprechende Virus-Sequenz das Target ist.

Erfindungsgemäße Antisense-PNA-Derivate, die gegen solche Targets wirksam sind, haben beispielsweise folgende Basensequenz.
a) gegen CMV, z. B.

| | |
|---|---|
| SEQ ID NO: 1 | 5'-GCGTTTGCTCTTCTTCTTGCG-3' |

b) gegen HIV, z. B.

| | |
|---|---|
| SEQ ID NO: 2 | 5'-ACACCCAATTCTGAAAATGG-3' |
| SEQ ID NO: 3 | 5'-AGGTCCCTGTTCGGGCGCCA-3' |

c) gegen HSV-1, z.B.

| | |
|---|---|
| SEQ ID NO:4 | 5'-GCGGGGCTCCATGGGGGTCG-3' |

Solche Arzneimittel eignen sich beispielsweise auch zur Behandlung von Krebs. Vorzugsweise können dabei Sequenzen zum Einsatz kommen, die gegen Targets gerichtet sind, die für Krebsentstehung bzw. Krebswachstum verantwortlich sind, insbesondere durch die Inhibition der Telomerase (E. Matthes et al. (1999) Nucleic Acids Res. 27, 1152). Solche Targets sind weiterhin:
1) Nucleare Onkoproteine wie beispielsweise c-myc, N-myc, c-myb, c-fos, c-fos/jun, PCNA, p120,
2) Cytoplasmische/Membran-assoziierte Onkoproteine wie beispielsweise EJras, c-Ha-ras, N-ras, rrg, bcl-2, cdc-2, c-raf-1, c-mos, c-src, c-abl, c-ets,
3) Zelluläre Rezeptoren wie beispielsweise EGF-Rezeptor, Her-2, c-erbA, VEGF-Rezeptor (KDR-1), Retinoid-Rezeptoren, Protein-Kinase regulatorische Untereinheit, c-fms, Tie-2, c-raf-1 Kinase, PKC-alpha, Protein Kinase A (R1 alpha),
4) Cytokine, Wachstumsfaktoren, Extrazelluläre Matrix wie beispielsweise CSF-1, IL-6, IL-1a, IL-1b, IL-2, IL-4, IL-6, IL-8, bFGF, VEGF, Myeloblastin, Fibronectin.

Antisense-PNA-Derivate, die gegen solche Targets wirksam sind, haben beispielsweise folgende Basen-Sequenz:
a) gegen c-Ha-ras, z. B.

| | |
|---|---|
| SEQ ID NO: 5 | 5'-CAGCTGCAACCCAGC-3' |
| SEQ ID NO: 6 | 5'-TATTCCGTCAT-3' |
| SEQ ID NO: 7 | 5'-TTCCGTCATCGCTCCTCAGGGG-3' |

b) bFGF, z.B.

| | |
|---|---|
| SEQ ID NO:8 | 5'-GGCTGCCATGGTCCC-3' |

c) c-myc, z.B.

| | |
|---|---|
| SEQ ID NO: 9 | 5'-GGCTGCTGGAGCGGGGCACAC-3' |
| SEQ ID NO: 10 | 5'-AACGTTGAGGGGCAT-3' |

d) c-myb, z.B.

| | |
|---|---|
| SEQ ID NO: 11 | 5'-GTGCCGGGGTCTTCGGGC-3' |

e) c-fos, z.B.

| | |
|---|---|
| SEQ ID NO:12 | 5'-CGAGAACATCATCGTGG-3' |
| SEO ID NO:13 | 5'-GGAGAACATCATGGTCGAAAG-3' |
| SEQ ID NO:14 | 5'-CCCGAGAACATCATGGTCGAAG-3' |
| SEQ ID NO:15 | 5'-GGGGAAAGCCCGGCAAGGGG-3' |

f) p120, z. B.

| | |
|---|---|
| SEQ ID NO: 16 | 5'-CACCCGCCTTGGCCTCCCAC-3' |

g) EGF-Rezeptor, z.B.

| | |
|---|---|
| SEQ ID NO: 17 | 5'-GGGACTCCGGCGCAGCGC-3' |
| SEO ID NO: 18 | 5'-GGCAAACTTTCTTTTCCTCC-3' |

h) p53 Tumorsuppressor, z.B.

| | |
|---|---|
| SEQ ID NO: 19 | 5'-GGGAAGGAGGAGGATGAGG-3' |
| SEQ ID NO: 20 | 5'-GGCAGTCATCCAGCTTCGGAG-3' |

i) bcl-2, z. B.

| | |
|---|---|
| SEQ ID NO: 21 | 5'-TCTCCCAGCGTGCGCCAT-3' |

k) VEGF, z. B.

| | |
|---|---|
| SEQ ID NO: 22 | 5'-GCGCTGATAGACATCCATG-3' |
| SEQ ID NO: 23 | 5'-GGAGGCCCGACC-3' |
| SEQ ID NO: 24 | 5'-GGTTTCGGAGGC-3' |
| SEQ ID NO: 25 | 5'-TGGTGGAGGTAG-3' |
| SEQ ID NO: 26 | 5'-GCATGGTGGAGG-3' |
| SEQ ID NO: 27 | 5'-TTGGCATGGTGG-3' |
| SEQ ID NO: 28 | 5'-GCCTGGGACCAC-3' |
| SEQ ID NO:29 | 5'-CAGCCTGGGACC-3' |
| SEQ ID NO:30 | 5'-TGCAGCCTGGGA-3' |
| SEQ ID NO:31 | 5'-GTGCAGCCTGGG-3' |
| SEQ ID NO:32 | 5'-GGTGCAGCCTGG-3' |
| SEQ ID NO:33 | 5'-ATGGGTGCAGCC-3' |
| SEQ ID NO:34 | 5'-GGCTTGAAGATG-3' |
| SEQ ID NO:35 | 5"-GCAGCCCCCGCA-3' |
| SEQ ID NO:36 | 5'-GCAGCAGCCCCC-3' |

l) c-raf Kinase, z. B.

| | |
|---|---|
| SEQ ID NO: 37 | 5'-TCCCGCCTGTGACATGCATT-3' |

m) PKC-alpha, z. B.

| | |
|---|---|
| SEQ ID NO: 38 | 5'-GTTCTCGCTGGTGAGTTTCA-3' |

n) Protein Kinase A, z. B.

| | |
|---|---|
| SEQ ID NO: 39 | 5'-GCGTGCCTCCTCACTGGC-3' |

Arzneimittel enthaltend PNA-Derivate der Formel I eignen sich beispielsweise ferner zur Behandlung von Erkrankungen, die durch Integrine oder Zell-Zell-Adhäsionsrezeptoren beeinflußt werden, beispielsweise durch VLA-4, VLA-2, ICAM, VCAM oder ELAM.

Antisense-PNA-Derivate, die gegen solche Targets wirksam sind, haben beispielsweise folgende Basen-Sequenz:
a) VLA-4, z. B.

| | |
|---|---|
| SEQ ID NO: 40 | 5'-GCAGTAAGCATCCATATC-3' |

b) ICAM-1, z. B.

| | |
|---|---|
| SEQ ID NO: 41 | 5'-GCCCAAGCTGGCATCCGTCA-3' |
| SEQ ID NO: 42 | 5'-CCCCCACCACTTCCCCTCTC-3' |
| SEQ ID NO: 43 | 5'-CTCCCCCACCACTTCCCCTC-3' |
| SEQ ID NO: 44 | 5'-GCTGGGAGCCATAGCGAGG-3' |

c) ELAM-1, z. B.

| | |
|---|---|
| SEQ ID NO:45 | 5'-ACTGCTGCCTCTTGTCTCAGG-3' |
| SEQ ID NO:46 | 5'-CAATCAATGACTTCAAGAGTTC-3' |

d) Integrin alpha(V), z. B.

| | |
|---|---|
| SEQ ID NO: 47 | 5'-GCGGCGGAAAAGCCATCG-3' |

Arzneimittel enthaltend PNA-Derivate der Formel I eignen sich beispielsweise auch zur Verhinderung der Restenose. Dabei können PNA-Sequenzen zum Einsatz kommen, die gegen Targets gerichtet sind, die für Proliferation oder. Migration verantwortlich sind. Solche Targets sind beispielsweise:
1) Nucleare Transaktivator-Proteine und Cycline wie beispielsweise c-myc, c-myb, c-fos, c-fos/jun, Cycline und cdc2-Kinase
2) Mitogene oder Wachstumsfaktoren wie beispielsweise PDGF, bFGF, VEGF, EGF, HB-EGF und TGF-β.
3) Zelluläre Rezeptoren wie beispielsweise bFGF-Rezeptor, EGF-Rezeptor und PDGF-Rezeptor.

Antisense-PNA-Derivate, die gegen solche Targets wirksam sind, haben beispielsweise folgende Basen-Sequenz:
a) c-myb, z. B.

| | |
|---|---|
| SEQ ID NO: 48 | 5'-GTGTCGGGGTCTCCGGGC-3' |

b) c-myc, z. B.

| | |
|---|---|
| SEQ ID NO: 49 | 5'-CACGTTGAGGGGCAT-3' |

c) cdc2-Kinase, z. B.

| | |
|---|---|
| SEQ ID NO: 50 | 5'-GTCTTCCATAGTTACTCA-3' |

d) PCNA (proliferating cell nuclear antigen of rat), z. B.

| | |
|---|---|
| SEQ ID NO: 51 | 5'-GATCAGGCGTGCCTCAAA-3' |

PNA-Derivate können ebenfalls zur Behandlung von Vitiligo und anderen Depigmentierungskrankheiten bzw. Depigmentierungsstörungen (z.B. der Haut, Haare, Augen) wie beispielsweise Albinismus und Psoriasis verwendet werden, oder zur Behandlung von Asthma, wobei die Expression des Adenosin-A1-Rezeptors, Adenosin-A3-Rezeptors, Bradikinin-Rezeptors oder von IL-13 mit Hilfe geeigneter Antisense Agenzien inhibiert werden. Eine solche Basensequenz ist beispielsweise:

| | |
|---|---|
| SEQ ID NO:52 | 5'-GATGGAGGGCGGCATGGCGGG-3' |

Arzneimittel enthaltend ein PNA-Derivat der Formel I können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talg und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.
Bevorzugte Verabreichungsformen sind topische Applikationen, lokale Applikationen wie beispielsweise mit Hilfe eines Katheters oder durch Inhalation, Injektionen bzw. Infusionen und die orale Verabreichung. Zur Injektion werden die PNA-Derivate der Formel I in einer flüssigen Lösung, vorzugsweise in einem physiologisch annehmbaren Puffer, wie z.B. Hank's Lösung oder Ringer's Lösung, formuliert. Die Oligonucleotide können aber auch in fester Form formuliert werden und vor dem Gebrauch gelöst oder suspendiert werden. Die für die systematische Verabreichung bevorzugten Dosierungen betragen ca. 0,01 mg/kg bis ca. 50 mg/kg Körpergewicht und Tag.

Die Erfindung betrifft weiterhin pharmazeutische Zubereitungen, die PNA-Derivate der Formel I und/oder deren physiologisch verträgliche Salze neben pharmazeutisch einwandfreien Träger- und/oder Zusatzstoffen enthalten.
Die PNA-Derivate der Formel und/oder deren physiologisch verträglichen Salze können am Tier, bevorzugt am Säugetier, und insbesondere am Menschen als Arzneimittel für sich allein, in Mischungen untereinander oder in Form von pharmazeutischen Zubereitungen verabreicht werden, die eine topische, perkutane, parenterale oder enterale Anwendung gestatten und die als aktiven Bestandteil eine wirksame Dosis mindestens eines PNA-Derivats, neben üblichen pharmazeutisch einwandfreien Träger- und Zusatzstoffen enthalten. Die Zubereitungen enthalten normalerweise etwa 0,1 bis 90 Gew.-% der therapeutisch wirksamen Verbindung. Zur Behandlung von Hautkrankheiten wird eine topische Anwendung, z.B. in Form von Salben, Lotionen oder Tinkturen, Emulsionen, Suspensionen bevorzugt.

Die Herstellung der pharmazeutischen Präparate erfolgt in an sich bekannter Weise, (z. B. Remingtons Pharmaceutical Sciences, Mack Publ. Co., Easton, PA.), wobei pharmazeutisch inerte anorganische und/oder organische Trägerstoffe verwendet werden. Für die Herstellung von Pillen, Tabletten, Dragees und Hartgelatinekapseln kann man z.B. Lactose, Maisstärke und/oder Derivate derselben, Talk, Stearinsäure und/oder deren Salze etc. verwenden. Trägerstoffe für Weichgelatinekapseln und/oder Suppositorien sind z.B. Fette, Wachse, halbfeste und flüssige Polyole, natürliche und/oder gehärtete Öle etc. Als Trägerstoffe für die Herstellung von Lösungen und/oder Sirupen eignen sich z.B.Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate und/oder Rods eignen sich Mischpolymerisate aus Glykolsäure und Milchsäure. Darüber hinaus sind Liposomenformulierungen, die dem Fachmann bekannt sind, geeignet (N. Weiner, Drug Develop Ind Pharm 15 (1989) 1523; "Liposome Dermatics, Springer Verlag 1992), beispielsweise HVJ-Liposomen (Hayashi, Gene Therapy 3 (1996) 878). Die dermale Applikation kann beispielsweise auch auch unter Zuhilfenahme ionophoretischer oder Methoden und/oder mit Hilfe der Elektroporation erfolgen.

Eine pharmazeutische Zubereitung kann neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel und/oder Lösungsvermittler und/oder Mittel zur Erzielung eines Depoteffekts, sowie Salze zur Veränderung des osmotischen Drucks, Überzugsmittel und/oder Antioxidantien enthalten. Sie können auch zwei oder mehrere verschiedene PNA-Derivate der Formel I und/oder deren physiologisch verträgliche Salze enthalten sowie ferner neben mindestens einem PNA-Derivate der Formel I einen oder mehrere andere therapeutisch wirksame Stoffe. Die Dosis kann innerhalb weiter Grenzen variieren und ist in jedem einzelnen Fall den individuellen Gegebenheiten anzupassen.

Weiterhin betrifft die Erfindung die Verwendung von PNA-Derivaten der Formel I als Diagnostikum, insbesondere als Hilfsmittel in der DNA-Diagnostik und in der Molekularbiologie (siehe zum Beispiel: Peptide Nucleic Acids: Protocols and Applications; Peter E. Nielsen und Michael Egholm (Edit.) Horizon Scientific Press, 1999). In der DNA-Diagnostik spielen Gensonden, auch DNA-Sonden oder Hybridization-Sonden genannt, eine große Rolle zum sequenzspezifischen Nachweis bestimmter Gene. Eine Gensonde besteht im allgemeinen aus einer Erkennungssequenz und einer bzw. mehreren geeigneten Markergruppen (Labeln). Die Spezifität der Bestimmung einer Targetsequenz in einer Analysenprobe mittels Hybridisierung mit einer komplementären Gensonde wird durch die Erkennungssequenz und deren chemische Struktur determiniert. Diese Technik kann auf PNA übertragen werden. Die PNA hat gegenüber den Oligonucleotiden natürlicher Struktur den Vorteil, dass sie eine höhere Affinität zur Targetsequenz und eine höhere Fähigkeit zur Basendiskriminierung aufweist.

Die Anwendung der Verbindungen der Formeln I bezieht sich daher auch auf die in-situ-Hybridisierung und Fluoreszenz-in-situ-Hybridisierung (FISH). Die in-situ-Hybridisierung kann beispielsweise auch zum Nachweis von Mikroorganismen und Viren dienen (Just et al. (1998) J. Vir. Method. 73, 163-174). Eine weitere Anwendung der erfindungsgemäßen Verbindungen bezieht sich zum Nachweis und Quantifizierung von Nukleinsäuren. Hierzu bedient man sich bevorzugt auch der Array-Technologie (Strother et al. J. Am. Chem. Soc. (2000) 122, 1205-1209; Niemeyer et al., Angew. Chem. (1999) 111, 3039-3043; Pirrung (1997) Chem. Rev. 97, 473-488), die einen hohen Probendurchsatz und hohe Empfindlichkeit aufweist. Dabei werden die PNA-Sonden auf einem geeigneten Träger oder PNA-Chip fixiert. Hierzu kann PNA wie in den Beispielen beschrieben synthetisiert werden und nachfolgend auf den Träger oder PNA-Chip fixiert werden. Alternativ kann die PNA direkt auf dem Träger hergestellt werden. Eine weitere Anwendung ist der Einsatz der Verbindungen der Formel I als Biosensoren zur Detektion von Nukleinsäuren (Wang et al (1996) J. Am. Chem. Soc. 118, 7667). Der Einsatz von PNA der Formel I mit einem Affinitätslabel, wie beispielsweise Histidyl-PNA, ist eine weitere Anwendung zur Reinigung von Nukleinsäuren (Oerum et al. (1999), in Peptide Nucleic Acids: Protocols and Applications).

Die beiden Phosphorylreste am Amino- und Carboxy-Terminus können unterschiedliche Funktionen erfüllen. Beispielsweise kann der Amino-Terminus zur Erhöhung der zellulären Aufnahme lipophil substituiert sein, während sich am Carboxy-Terminus ein Fluorescein-Rest zum Nachweis der verbesserten Zellaufnahme befindet (vgl. PNA-6 in Beispiel 7).

Die erfindungsgemässen PNA Derivate eignen sich somit auch als Antisense-, Anti-gen, Decoy oder Chimeraplasty-Agenzien.

Die zweifach derivatisierten Verbindungen der Formel I eignen sich auch als sogenannte "Molecular Beacons" (Li et al. (2000) Angew. Chemie 112, 1091-1094), die erst bei der Bindung an eine komplementäre Nukleinsäure ein Fluoreszenzsignal abgeben. In diesen Beacons ist ein Ende der PNA, beispielsweise der Amino-Terminus, mit einem Fluoreszenz-Label versehen, während das andere Ende, beispielsweise der Carboxy-Terminus, mit einem Quencher versehen ist. Auch der umgekehrte Fall, in dem der N-Terminus einen Quencher trägt, und der C-Terminus ein Fluoreszenz-Label, ist möglich. Dadurch kommt es zur Unterdrückung des Fluoreszenzsignals, solange das zweifach markierte PNA-Derivat nicht an eine komplementäre Nukleinsäure bindet. Erst bei der Bindung werden der Fluoreszenz-Rest (z.B. Edans) und der Quenscher (z. B. Dabcyl) räumlich voneinander getrennt, so dass ein Fluoreszenzsignal ausgesandt wird (Sokol et al. (1998) Proc. Natl. Acad. Sci. 95, 11538).

Die Synthese des PNA-Rückgrates erfolgt nach den in der Literatur beschriebenen Methoden, zum Beispiel nach der tert-Butyloxycarbonyl- (BOC), 9-Fluorenylmethoxycarbonyl- (Fmoc) oder Monomethoxytrityl- (Mmt) Schutzgruppen-Strategie (Peptide Nucleic Acids: Protocols and Applications; Peter E. Nielsen und Michael Egholm (Edit.) Horizon Scientific Press, 1999). Vorzugsweise wird die Mmt-Schutzgruppe zum temporären Schutz der Aminofunktion des Aminoethylglycins verwendet und basenlabile Schutzgruppen an den heterozyklischen Nucleobasen (D. Will et al. (1995) Tetrahedron 51, 12069; Breipohl et al. (1997) Tetrahedron 53, 14671-14686). Beispiele für Monomerbausteine sind Verbindungen der Formel V bis V D, wobei A, B, D, E, u' und V' obige Bedeutung haben, PG eine Aminosäure-Schutzgruppe wie beispielsweise Benzoyl, Anisoyl-, Isobutyroyl-, Acetyl-, tert-Butylbenzoyl (Breipohl et al. (1997) Tetrahedron 53, 14671-14686) ist, TR eine säurelabile Schutzgruppe wie Dimethoxytrityl (Dmt) (für V' = O und S) oder Mmt (für V' = NH) ist.

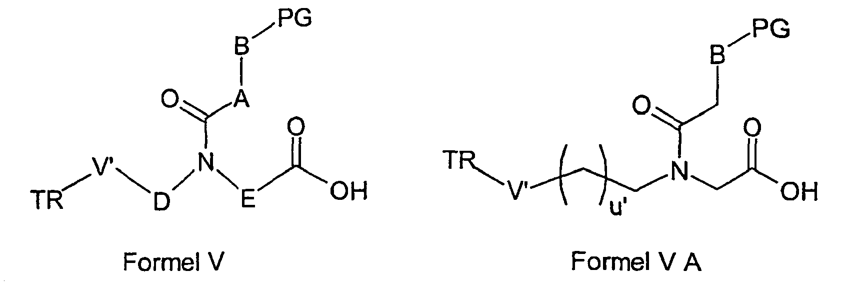

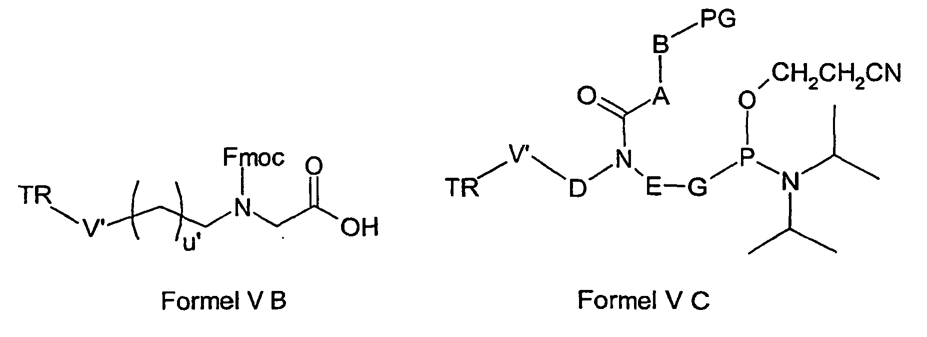

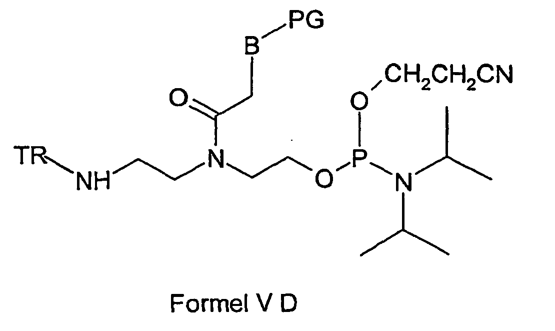

Nach Aufbau des PNA-Rückgrates kann die freie Aminofunktion des N-Terminus direkt mit einem entsprechenden Phosphorylierungsreagenz beispielsweise zu einem Phosphoramidat (V' = NR₁ in Formel I) umgesetzt werden.

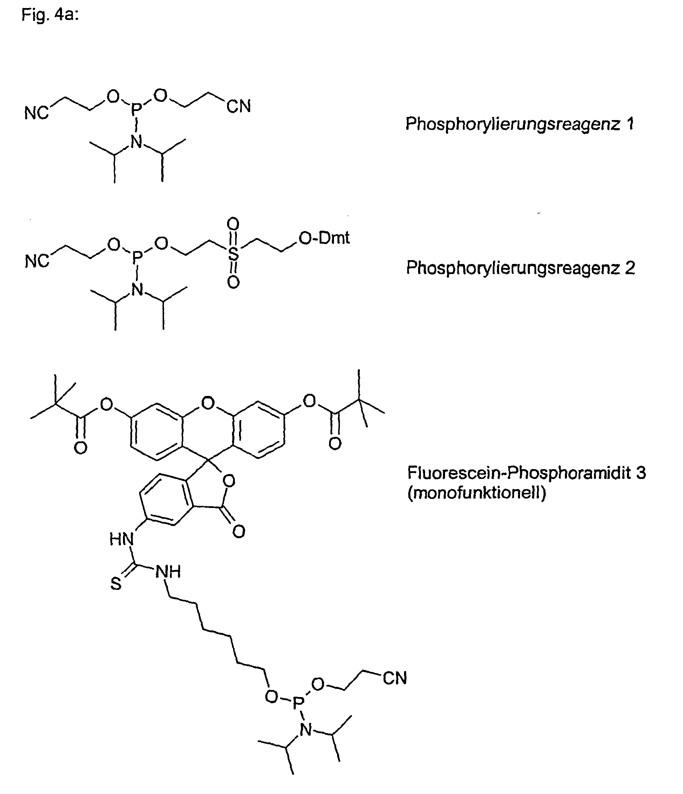

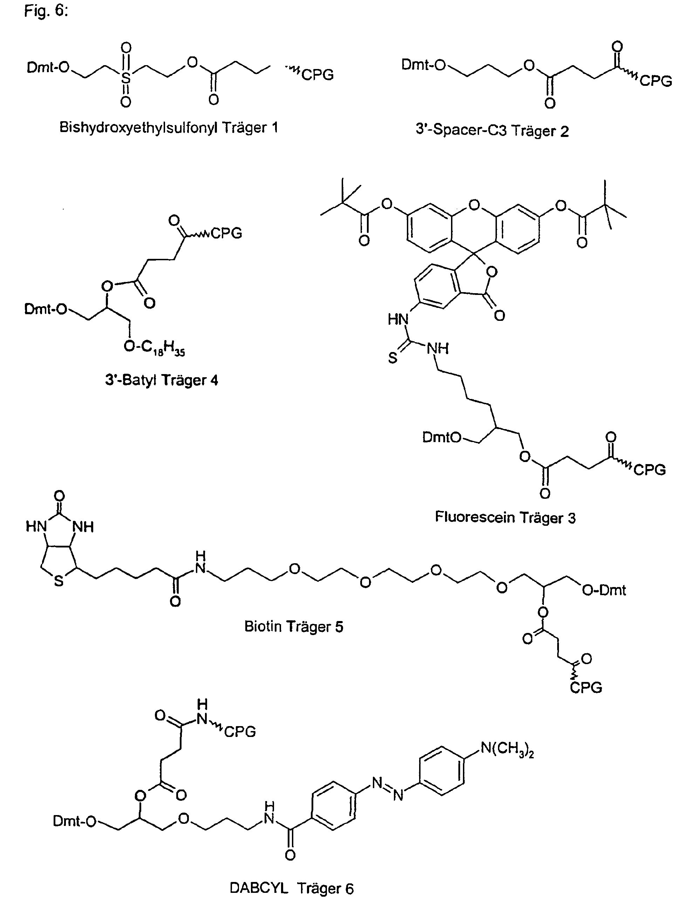

Die Phosphoryl-Reste können mit Hilfe von in der Nucleotid-Chemie üblicherweise verwendeten Reagenzien eingeführt werden. Es sind zahlreiche Phosphorylierungsreagenzien zugänglich, die zur Herstellung der Verbindungen der Formel I herangezogen werden können. Eine Auswahl der Reagenzien ist in den Figuren 4a bis 4d gezeigt, wobei die Erfindung aber nicht auf diese spezielle Derivate beschränkt ist. Für die carboxyterminale Modifikation kommen entsprechend modifizierte Träger, insbesondere CPG-Träger für die Festphasensynthese zum Einsatz. Beispiele solcher Träger sind in Figur 6 aufgeführt.

Als Phosphorylierungsreagenzien können die in der Nucleotidchemie üblichen Reagenzien eingesetzt werden (Glen Research Corporation, Sterling, VA 20164, U.S.A.; Figur 4a bis 4d), die beispielsweise nach der Phosphoramidit-Methode, der H-Phosphonat-Methode oder der Phosphotriester-Methode reagieren (E. Sonveaux (1986) Bioorganic Chemistry 14, 274; S. L. Beaucage und R. P. lyer (1993) Tetrahedron 49, 1925; E. Uhlmann und A. Peyman (1990) Chem. Rev. 90, 543). Die Vielfalt der möglichen Modifikationen ist durch die große Zahl der bekannten Phosphorylierungsreagenzien und entsprechend derivatisierter Träger, insbesondere von controlled pore glass (CPG) Trägem, bestimmt. Als feste Träger werden ausserdem bevorzugt tentagel® (Fa. Rapp Polymers GmbH, Tübingen) und Aminomethylpolystyren benutzt.

Zur Einführung der Phosphoryl-Funktion kommen im Prinzip alle in der Nucleotidchemie bekannten Reagenzien in Betracht, insbesondere aber die folgenden Reagenzien der Formel VI A, Formel VI B, Formel VI C und Formel VI D, 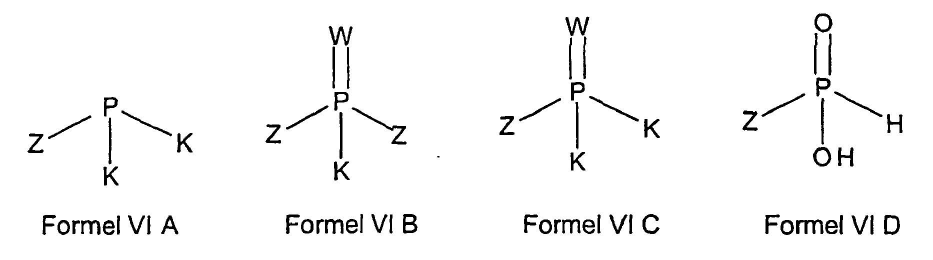 wobei K gleich Halogen, bevorzugt Cl, Triazolyl, Imidazolyl, oder Dialkylamino ist, W die oben genannte Bedeutung oder die. Bedeutung von W' haben kann, und Z die oben genannte Bedeutung oder die Bedeutung von X, X' oder Z' haben kann, wobei reaktive Gruppen entsprechend geschützt sind.

Beispielsweise sind die Hydroxygruppen des Fluorescein-Phosphoramidits 3 (Figur 4a) durch Veresterung mit Pivalinsäure geschützt.

Die Verbindungen der Formel VI sind nur als Beispiele für solche Reagenzien zu sehen, die gegebenenfalls unter Zugabe weiterer Hilfsreagenzien wie Basen, Säuren oder Kondensationsreagenzien reagieren. Besonders bevorzugt sind die Reagenzien der Formel VI A, die nach der Phosphoramidit-Methode reagieren (Beaucage und lyer, 1993). Diese werden als Phosphor-(III)-Verbindung zur Reaktion gebracht und anschließend oxidiert. Wird die Oxidation beispielsweise mit Jod/Wasser/Pyridin oder tert-Butylhydroperoxid durchgeführt, erhält man die Phosphorylderivate (W = O). Erfolgt die Oxidation dagegen mit elementarem Schwefel oder Beaucage-Reagenz, so erhält man die entsprechende Thiophosphoryl-Verbindung (W = S).

Unter den Reagenzien (Figuren 4a bis 4d) befinden sich auch "bifunktionelle Reagenzien", die aufgrund einer zweiten Funktion, welche zunächst geschützt ist, mehrfach zur Reaktion gebracht werden können. Beispiele für solche bifunktionellen Reagenzien sind die Phosphoramidite 4, 6, 8 bis 13. Dabei kann es sich um die multiple Konjugation eines Reagenzes oder aber um die sukzessive Reaktion mit unterschiedlichen Reagenzien handeln. So kann beispielsweise das Fluorescein-Phosphoramidit 3 nur einmal zur Reaktion gebracht werden. Dagegen besitzt das Fluorescein-Phosphoramidit 4 eine durch eine Dmt-Gruppe geschützte Hydroxyfunktion, die nach Abspaltung der Dmt-Gruppe nochmals mit einem Phosphorylierungsreagenz zur Reaktion gebracht werden kann. Auf diese Weise können ein und dieselbe Gruppe oder aber unterschiedliche Gruppen mehrfach eingeführt werden. PNA-6 ist ein Beispiel für eine Mehrfachkonjugation am Carboxyterminus und einer zusätzlichen Modifikation am Aminoterminus. Zunächst wurden am Carboxy-terminus sukzessive das Fluorescein und der Aminolinker aufgebaut. Nach der Synthese des PNA-Teifs wurde im letzten Zyklus ein Hydroxyethylglycin-t Baustein gekoppelt, der mit dem C16-Phosphorylierungsreagenz 7 zur Reaktion wurde. PNA-1 und PNA-2 sind Verbindungen der Formel I, die nur carboxyterminal mit einem Phosphoryl-Rest modifiziert sind (q = 0). Diese Substanzklasse ist ebenfalls neu und Gegenstand der Erfindung.

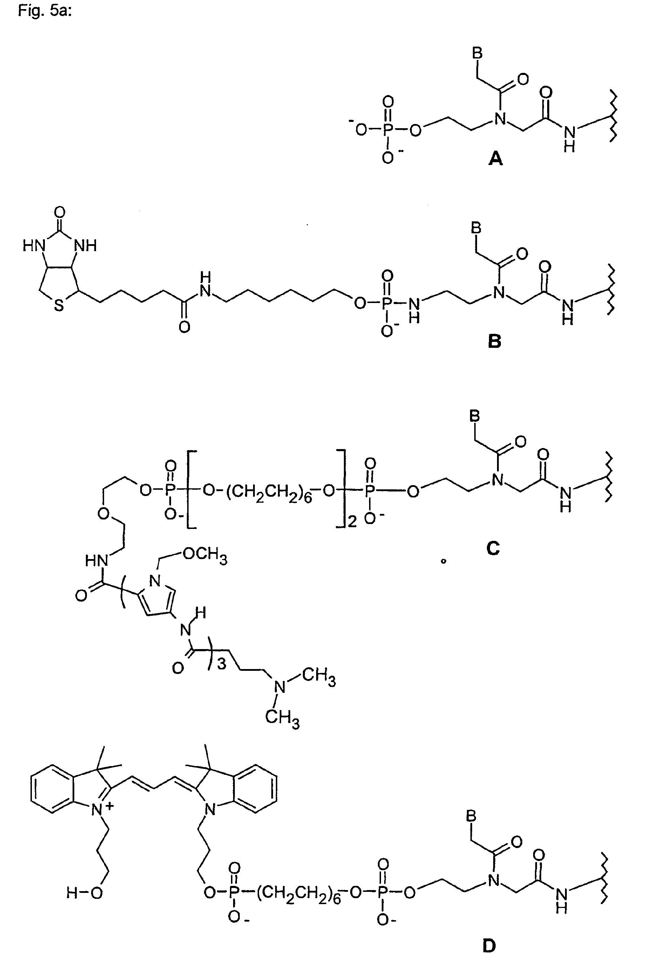

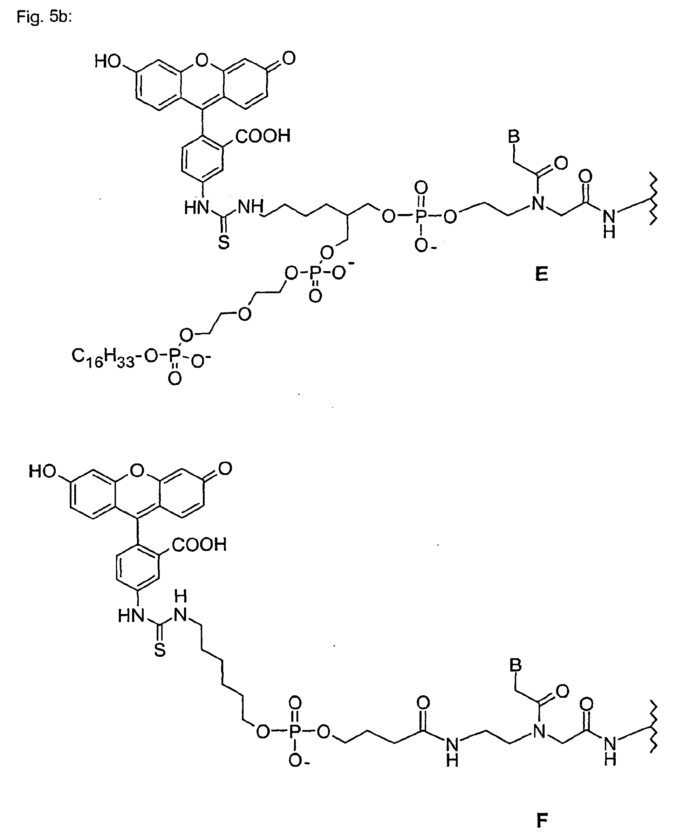

In Figur 5a und 5b sind einige Beispiele von Verbindungstypen für die N-terminale Modifikation der Verbindungen der Formel I gezeigt. Verbindungstyp A erhält man durch Reaktion der endständigen Hydroxygruppe der PNA mit dem Phosphorylierungsreagenz 1. Verbindungstyp B erhält man durch Reaktion der endständigen Aminogruppe der PNA mit dem Biotin-Phosphoramidit 5. Verbindungstyp C erhält man durch sukzessive Umsetzung der PNA mit einer endständigen Hydroxygruppe mit dem Spacer-18 Phosphoramidit 9, Aminomodifier-5 Phosphoramidit 12 und Lexitropsin. Verbindungstyp D erhält man durch sukzessive Umsetzung der PNA mit einer endständigen Hydroxygruppe mit dem Spacer-9 Phosphoramidit 8 und dem Cyanin-3 Phosphoramidit 10. Verbindungstyp E erhält man durch sukzessive Umsetzung der PNA mit einer endständigen Hydroxygruppe mit dem bifunktionellen Fluorescein-Phosphoramidit 4, dem Spacer-9 Phosphoramidit 8, und dem C16-Phosphorylierungsreagenz 7. Die zusätzlich durchzuführenden Schritte, wie Oxidiation und Schutzgruppenabspaltung, sind in den Beispielen beschrieben.

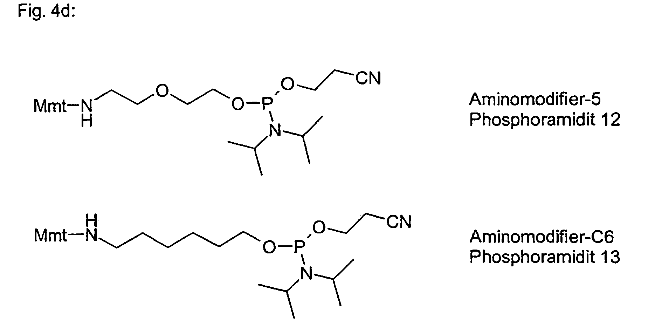

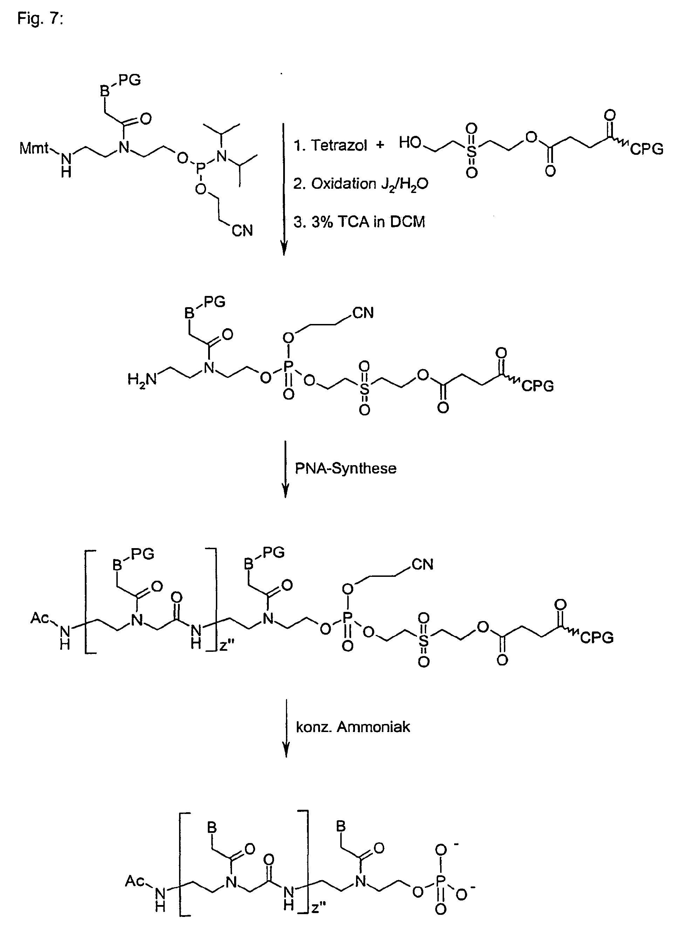

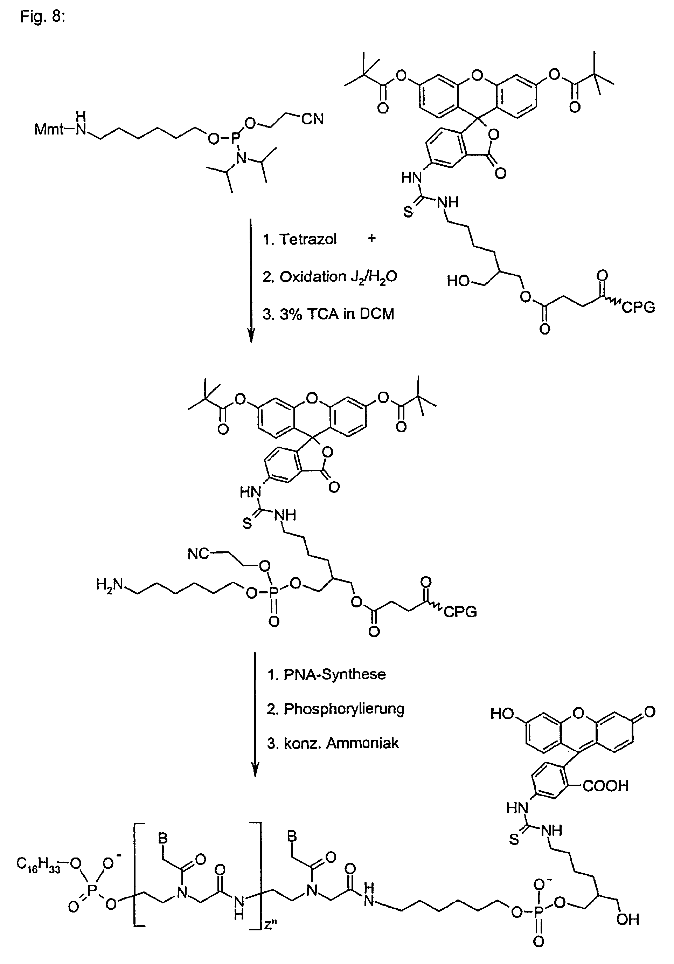

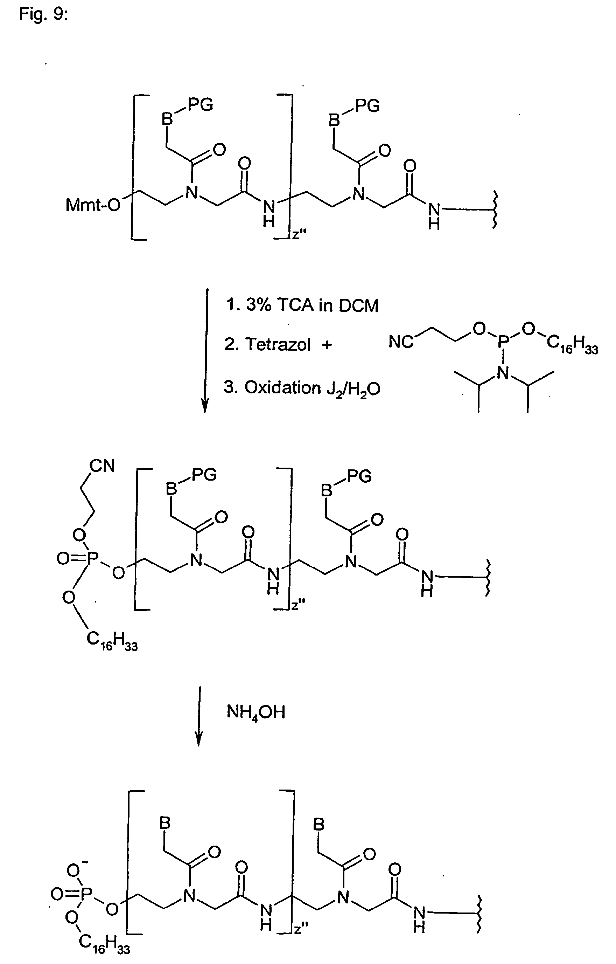

Ein Beispiel für eine carboxyterminale Modifikation von PNA mit Hilfe eines Phosphoramidits der Formel V D ist in Figur 7 dargestellt. Dabei geht man von einem Bishydroxyethylsulfon-Träger 1 (Figur 6) aus, der nach Abspaltung der Dmt-Gruppe mit 3% Trichloressigsäure mit dem Phosphoramidit der Formel V D unter Tetrazolkatalyse umgesetzt wird. Nach Oxidation mit Jodwasser spaltet man die aminoterminale Mmt-Gruppe mit 3% Trichloressigsäure ab und synthetisiert dann den PNA-Teil nach literaturbekannten Methoden, beispielsweise nach der unten erläuterten Mmt-Methode. Eine alternative Methode zur carboxyterminalen Modifikation macht von CPG®-Trägern Gebrauch, die entsprechend dem einzuführenden Rest modifiziert sind, also beipielsweise den Fluorescein-Rest enthalten (Figur 8). Diese Methode soll am Beispiel eines PNA-Derivates erläutert werden, das aminoterminal mit einem Hexadecylphosphat-Rest und carboxyterminal mit einem Fluoresceinphosphat modifiziert ist. Zunächst wird der Fluorescein-Träger 3 (Figur 6) mit Trichloressigsäure detrityliert und dann mit dem Aminomodifier-C6 Phosphoramidit 13 (Figur 4d) mit Hilfe von Tetrazol kondensiert. Nach Oxidation mit Jodwasser und Abspaltung der Mmt-Gruppe kann der PNA-Teil nach gängigen Methoden synthetisiert werden. Im letzten Zyklus wird ein auf Hydroxyethylglycin basierender PNA-Baustein (Formel V A, u' = 2, V' = Sauerstoff) gekuppelt, der nach Abspaltung der Dmt-Schutzgruppe mit Hilfe des C16-Phosphorylierungsreagenzes 7 wie in Figur 9 gezeigt umgesetzt wird. Nach Abspaltung aller Schutzgruppen und Spaltung vom CPG-Träger erhält man das zweifach modifizierte PNA-Derivat.

### Beispiele:

Die Herstellung folgender Verbindungen ist exemplarisch beschrieben: 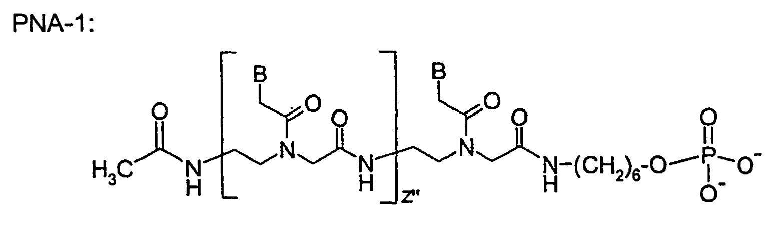 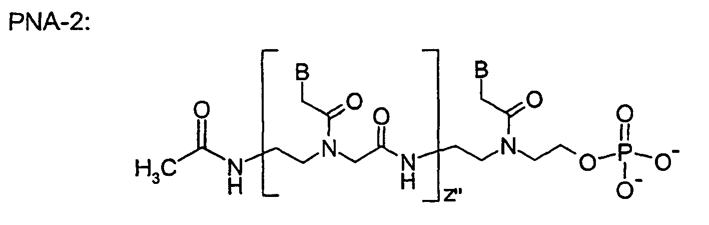 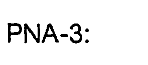 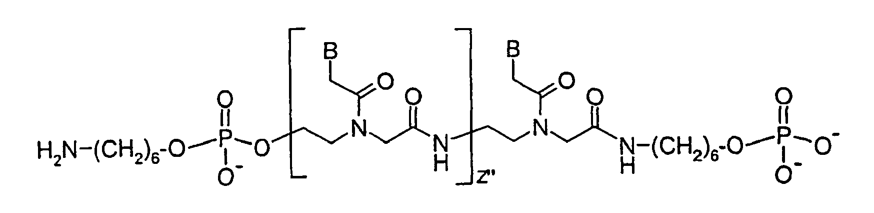 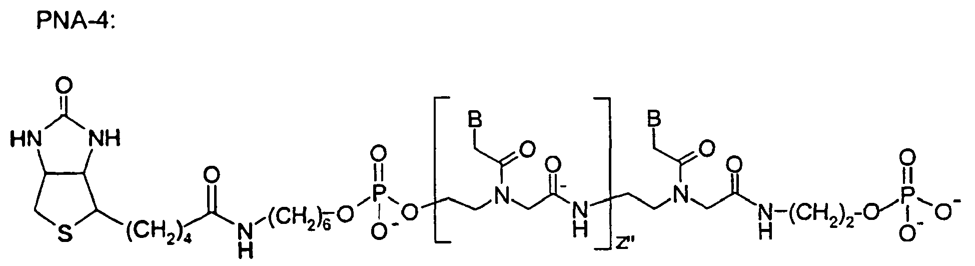 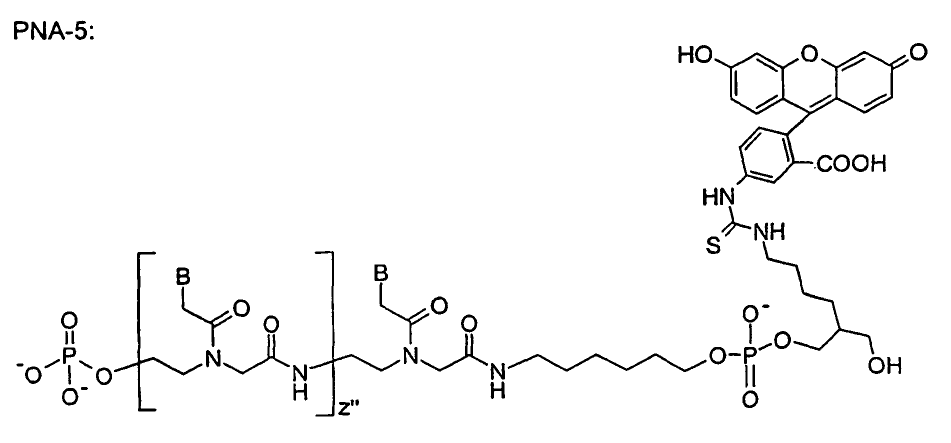 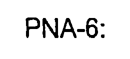 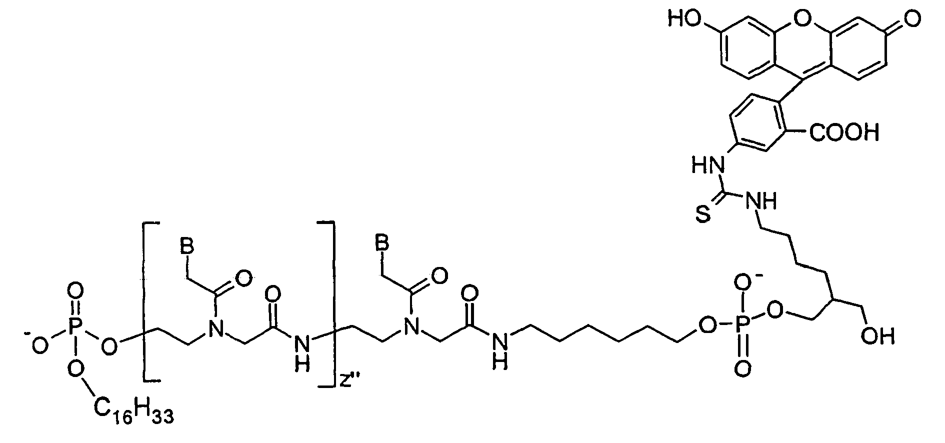 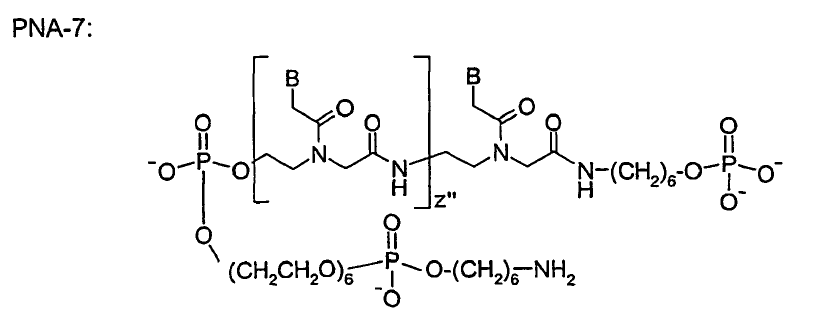 wobei die Abfolge der Basen B jeweils durch SEQ ID NO: 53 beschrieben wird, und z" jeweils gleich 10 ist:

| | | |
|---|---|---|
| SEQ ID NO: 53 | 5'-TATTCCGTCA T-3' | (PNA-1 bis PNA-7) |

### Beispiel 1: Synthese der PNA-Kette

Zur Herstellung des PNA-Teils wurden die folgenden Reagenzien verwendet:
1. Phosphoramidit-Reagenz (0.1 M in Acetonitril (ACN))
2. Mmt-PNA-Monomere bzw. Dmt-oeg-PNA-Monomere (0.2 M in DMF:ACN (1:1; v:v))
3. Wasserfreies ACN (≤ 30 ppm water)
4. Trichloressigsäure (3 %) in Dichloromethan (DCM)
5. Acetanhydrid, 2,6-Lutidin in THF (1:1:8; v:v:v); (Cap A)
6. N-Methylimidazol (16 %) in THF; (Cap B)
7. Jodlösung (0.05 M) in THF, Wasser, Pyridin, (7:2:1; v:v:v)
8. Waschlösung (THF, Wasser, Pyridin (7:2:1; v:v:v))
9. Tetrazol (0.3 M) in ACN
10. HBTU; 0.2 M in DMF:ACN (1:1; v:v)
11. DIPEA; 0.2 M in DMF:ACN (1:1; v:v)
12. DMF (> 99.5 %)
13. Festphasenträger: Aminopropyl-CPG® (550 Å) beladen mit Mmt-Aminohex-1-yl hemisuccinat (für PNA-hexylamide).

Die Mmt/Acyl-geschützten bzw. Dmt/Acyl-geschützten oeg-Monomere wurden wie bereits beschrieben hergestellt (Breipohl et al. (1997) Tetrahedron 53, 14671-14686). Die Beladung von Aminopropyl-CPG® mit dem Mmt-Aminohex-1-yl-hemisuccinat wurde ebenfalls bereits beschrieben (Will et al. (1995) Tetrahedron 51, 12069-12082). Die derivatisierten CPG®-Träger sind kommerziell erhältlich (Glen Research Corporation, Sterling, VA 20164, U.S.A.). Die PNA-Synthesen wurden im allgemeinen im Massstab von 2 to 5 µmol durchgeführt.

Folgender Zyklus wurde zur PNA-Synthese verwendet:
1. Waschschritt mit ACN
2. Entschützung der Mmt-Gruppe bzw. Dmt-Gruppe durch Behandlung mit 3% TCA in DCM; 110 sec.
3. Waschschritt mit DMF/ACN (1:1)
4. Neutralisierung mit DIPEA in DMF/ACN (1:1)
5. Kupplung des Monomer-Bausteins durch Voraktivierung (15 min) mit HBTU/DIPEA/PNA-Monomer (Verhältnis 1:1:1; Gesamtvolumen 450 µl) Beschickung der Festphase und Kupplung (45 min)
6. Waschschritt mit ACN
7. Capping mit Acetanhydrid/N-Methylimidazol
8. Waschschritt mit ACN
9. Neuer Zyklus

### Beispiel 2: Synthese von acetyl-tat tcc gtc at-aminohexyl-p (PNA-1)

Zunächst wird vom Bishydroxyethylsulfonyl-Träger 1 (1µmol, Figur 6) durch Behandlung mit 3% Trichloressigsäure die Dmt-Schutzgruppe abgespalten. Dann wird die freie Hydroxyfunktion mit dem Aminomodifier-C6 Phosphoramidit 13 (Figur 4d) unter Tetrazol-Katalyse zur Reaktion gebracht. Dabei werden das Phosphorylierungsreagenz 13 im Überschuss (ca. 25-fach) als 0.3 M Lösung in Acetonitril/Tetrahydrofuran (1:1; v:v) und das Tetrazol (ca. 50-fach; 0.5 M in Acetonitril) eingesetzt. Nach erfolgter Kondensation wird mit einer Jodlösung (0.05 M in Tetrahydrofuran/ Wasser, Pyridin (7:2:1; v:v:v)) oxidiert. Danach wird der PNA-Teil wie in Beispiel 1 beschrieben durch Festphasensynthese hergestellt. Im letzten Zyklus wird die freie Aminofunktion durch Behandlung mit dem Capping-Reagenz acetyliert. Dies verhindert bei der Entschützung mit konz. Ammoniak den aminoterminalen Abbau der PNA. Schließlich wird die PNA durch Behandlung mit konz. Ammoniak bei 50°C über Nacht vom Träger gespalten und gleichzeitig die Schutzgruppen entfernt. Man erhält 103 OD (260) des gewünschten Rohprodukts, das durch präparative Polyacrylamid (PAA)-Gelektrophorese gereinigt wurde. Die gewünschte Produktbande wird mit 0.2M Triethylammoniumbikarbonat-Puffer eluiert und über eine Bond-Elut C18-Säule (1 g) entsalzt. Man erhält 23.3 OD. Das Produkt wurde durch Negativionen-Massenspektrometrie analysiert, welche die berechnete Masse bestätigte (ber. 3166.2; gef. 3166.8).

### Beispiel 3: Synthese von acetyl-tat tcc gtc at(eo)-p (PNA-2)

Die Herstellung erfolgt analog wie in Beispiel 2 beschrieben in einer 1 µmol Synthese. Nach Abspaltung der Dmt-Schutzgruppe des Trägers 1 (Figur 6) wird die freie Hydroxyfunktion mit dem Phosphoramidit der Formel V D unter Tetrazol-Katalyse zur Reaktion gebracht. Dabei werden das Phosphoramidit im Überschuss (ca. 20-fach) als 0.1 M Lösung in Acetonitril/Tetrahydrofuran (1:1; v:v) und das Tetrazol (ca.- 50-fach; 0.5 M in Acetonitril) eingesetzt. Nach erfolgter Kondensation wird mit einer Jodlösung (0.05 M in Tetrahydrofuran/Wasser, Pyridin (7:2:1; v:v:v)) oxidiert. Nach der Spaltung mit Ammoniak erhält man 50 OD Rohprodukt. Davon wurden 45 OD über ein 15% PAA Gel durch Elektrophorese gereinigt wird. Man erhält 13.2 OD Produkt mit dem Molekulargewicht 3052.9 (ber. 3052.9).

### Beispiel 4: Synthese von Aminohexyl-p-t(oeg) at tcc gtc at-aminohexyl-p (PNA-3)

Die Herstellung erfolgt analog wie in Beispiel 2 beschrieben in einer 1 µmol Synthese. Nach Aufbau des Carboxyterminus und Synthese des PNA-Teils wird im letzten Zyklus allerdings ein auf Hydroxyethylglycin basierender Baustein mit Thymin als Nucleobase (oegT) gekuppelt. Nach Spaltung der Dmt-Gruppe wird die freie Hydroxyfunktion mit dem Aminomodifier-C6 Phosphoramidit 13 (Figur 4d) unter Tetrazol-Katalyse gekuppelt und anschliessend mit Jodwasser oxidiert. Durch Behandlung mit konz. Ammoniak bei 50°C spaltet man das Oligomer vom Träger und entfernt gleichzeitig alle basenlabilen Schutzgruppen. Dann wird die terminale Mmt-Schutzgruppe durch Behandlung mit 80% Essigsäure entfernt. Man erhält 130 OD des Rohproduktes, das durch GelElektrophorese gereinigt wurde. Man erhält 22.5 OD Produkt mit dem Molekulargewicht 3303.8 (ber. 3305.0)

### Beispiel 5: Synthese von Biotin-p-t(oeg) at tcc gtc at-aminohexyl-p (PNA-4)

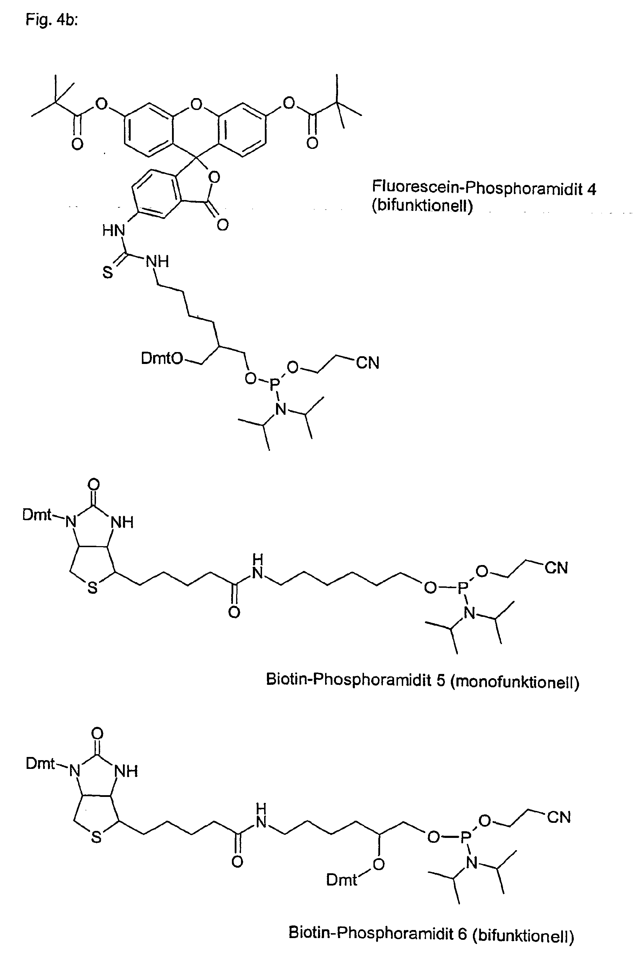

Die Herstellung erfolgt analog wie in Beispiel 2 beschrieben in einer 0.5 µmol Synthese. Nach Aufbau des Carboxyterminus und Synthese des PNA-Teils wird im letzten Zyklus allerdings ein auf Hydroxyethylglycin basierender Baustein mit Thymin als Nucleobase (oegT) gekuppelt. Nach Spaltung der Dmt-Gruppe wird die freie Hydroxyfunktion mit dem Biotin-Phosphoramidit 5 (Figur 4b) unter Tetrazol-Katalyse gekuppelt und anschliessend mit Jodwasser oxidiert und mit Trichloressigsäure detrityliert. Durch Behandlung mit konz. Ammoniak bei 50°C spaltet man das Oligomer vom Träger und entfernt gleichzeitig alle Schutzgruppen. Man erhält 37 OD des Rohproduktes, das durch GelElektrophorese gereinigt wurde. Man erhält 22.5 OD.

### Beispiel 6: Synthese von p-t(oeg) at tcc gtc at-aminohexyl-p-Fluorescein (PNA-5)

Die Synthese erfolgt analog Beispiel 2 ausgehend vom Fluorescein-Träger 3 (Figuren 6a und 8). Vom Fluorescein-Träger 3 wird durch Behandlung mit 3% Trichloressigsäure die Dmt-Schutzgruppe abgespalten. Dann wird die freie Hydroxyfunktion mit dem Aminomodifier-C6 Phosphoramidit 13 (Figur 4d) unter Tetrazol-Katalyse zur Reaktion gebracht. Nach erfolgter Kondensation wird mit einer Jodlösung (0.05 M in Tetrahydrofuran/ Wasser, Pyridin (7:2:1; v:v:v)) oxidiert. Danach wird der PNA-Teil wie in Beispiel 1 beschrieben durch Festphasensynthese hergestellt. Im letzten Zyklus wird ein auf Hydroxyethylglycin basierender Baustein mit Thymin als Nucleobase ((t)oeg) gekuppelt. Nach Spaltung der Dmt-Gruppe wird die freie Hydroxyfunktion mit dem Phosphorylierungsreagenz 1 (Figur 4a) unter Tetrazol-Katalyse gekuppelt und anschliessend mit Jodwasser oxidiert. Schliesslich wird die PNA durch Behandlung mit konz. Ammoniak bei 50°C über Nacht vom Träger gespalten und gleichzeitig die Schutzgruppen entfernt. Man erhält 61 OD (260) des Rohprodukts, das durch präparative Polyacrylamid (PAA)-Gelelektrophorese gereinigt wurde. Die gewünschte Produktbande wird mit 0.2M Triethylammoniumbikarbonat-Puffer eluiert und über eine Bond-Elut C18-Säule (1 g) entsalzt. Man erhält 5.6 OD. Das Produkt wurde durch Negativionen-Massenspektrometrie analysiert, welche die berechnete Masse zeigte (ber. 3709.5; gef. 3706.3)

### Beispiel 7: Synthese von C16-p-t(oeg) at tcc gtc at-aminohexyl-p-Fluorescein (PNA-6)

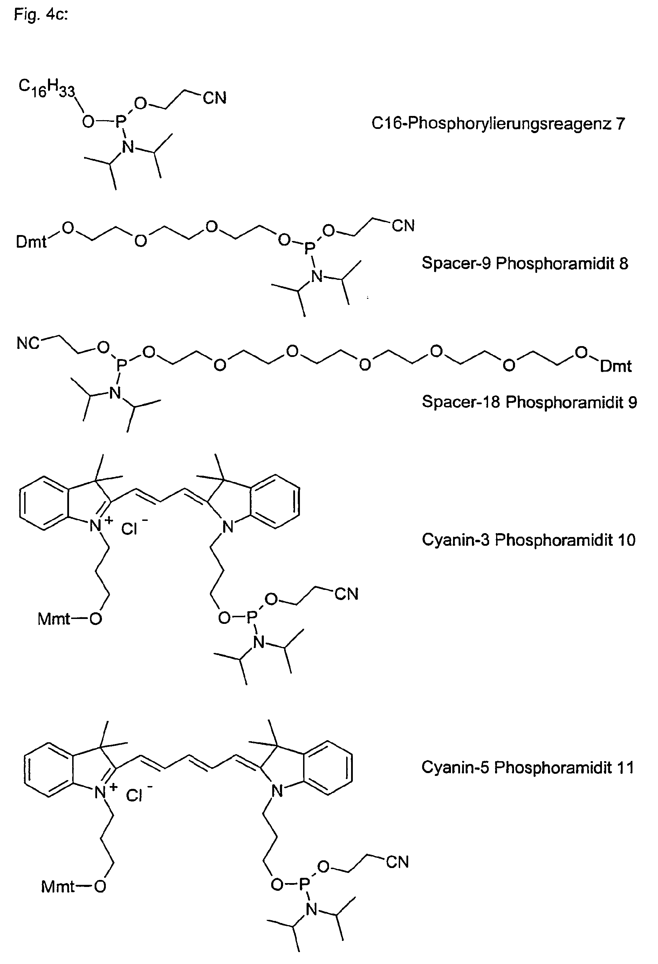

Die Synthese erfolgt analog Beispiel 6 ausgehend von 1 µmol Fluorescein-Träger 3 (Figuren 6a und 8). Im letzten Zyklus wurde ein auf Hydroxyethylglycin basierender Baustein mit Thymin als Nucleobase ((t)oeg) gekuppelt. Nach Spaltung der Dmt-Gruppe wird die freie Hydroxyfunktion jedoch mit dem C16-Phosphorylierungsreagenz 7 (Figur 4c) unter Tetrazol-Katalyse gekuppelt und anschliessend mit Jodwasser oxidiert. Schliesslich wird die PNA durch Behandlung mit konz. Ammoniak bei 50°C über Nacht vom Träger gespalten und gleichzeitig die Schutzgruppen entfernt. Man erhält 61 OD (260) des gewünschten Rohprodukts, das durch präparative Polyacrylamid (PAA)-Gelektrophorese gereinigt wurde. Die gewünschte Produktbande wird mit 0.2M Triethylammoniumbikarbonat-Puffer eluiert und über eine Bond-Elut C18-Säule (1 g) entsalzt. Man erhält 4.6 OD. Das Produkt wurde durch Negativionen-Massenspektrometrie analysiert, welche die berechnete Masse zeigte (ber. 3934, gef. 3931).

### Beispiel 8: Bestimmung der Schmelztemperaturen

Die Bestimmung der Schmelztemperaturen erfolgte mit Hilfe eines HP 8452A Diodenarray-Spektrophotometers, eines HP 89090A Peltier-Elements und der HP Temperature Control Software Rev. B5.1 (Fa. Hewlett Packard). Es wird in 0.5°C/min Schritten in 140 mM KCI, 10 mM Natriumdihydrogenphosphat, 0.1 mM EDTA (pH 7.4) als Puffer gemessen. Die Oligomerkonzentration beträgt 0.5 bis 1 OD₂₆₀ pro ml.

Überraschenderweise zeigten die zweifach phosphorylmodifzierten PNA-5 und PNA-6 Derivate mit zwei bzw. drei negativen Ladungen eine gleich gute oder bessere Bindung gegenüber komplementärer DNA und RNA wie die ungeladene PNA (Referenzsubstanz).

| PNA-Derivat | | Tₘ (DNA) | Tₘ (RNA) |
|---|---|---|---|
| Referenz | Ac-HN-tat tcc gtc at-hex | 41.9 °C | 56.6 °C |
| PNA-5 | p-t(oeg) at tcc gtc at-aminohexyl-p-Fluorescein | 41.8 °C | 56.9 °C |
| PNA-6 | C16-p-t(oeg) at tcc gtc at-aminohexyl-p-Fluorescein | 44.1 °C | 56.9 °C |

### Beispiel 9: Bestimmung der Zellaufnahme nach Fluoreszenz-Markierung

Man läßt die COS-Zellen bis zur Konfluenz in Dulbecco's MEM, das mit 10 % FCS supplementiert wurde, in 5 cm Petrischalen heranwachsen. Die Zellen werden zweimal mit serumfreien DMEM gewaschen. Mit Hilfe einer sterilen Nadel wird eine Fläche von ca. 1 cm² in der Mitte der Petrischale eingekratzt. In diese Fläche wird die zu untersuchende PNA-Lösung (10 µM) aufgebracht. Es wird bei 37 °C unter CO₂-Atmosphäre inkubiert. Nach 2, 4 und 16 Stunden werden die Zellen durch Fluoreszenzmikroskopie untersucht. Dazu werden die Zellen viermal mit serumfreien DMEM gewaschen, mit einem Glasträger abgedeckt und unter dem Fluoreszenzmikroskop bzw. durch Phasenkontrast bewertet. PNA-5 und PNA-6 wurden fluoreszenzmikroskopisch untersucht.

Dabei zeigte sich, dass das Hexadecyl-PNA-Derivat (PNA-6) effizienter in die Zellen aufgenommen wurde als die PNA ohne Hexadecyl-Rest.

### Beispiel 10: Hemmung der Zellproliferation durch PNA-6

Die Sequenz von PNA-6 ist gegen den Translationsstart der Ha-ras mRNA gerichtet. Die REH Zellen (human pre-B leukemia cells, DSM ACC 22) oder

### A549 Tumor zellen wurden in OptiMEM (Gibco BRL) mit 10 % fötalem

Kälberserum (FCS, GIBCO-BRL) bei 37°C unter 5% CO₂ kultiviert. Die Zelldichte für den Assay war ca. 1 x 106 / ml). Die PNA-6 (10 µM) wurde mit den Zellen in 24-well Platten inkubiert. Nach 96 Inkubation bei 37°C unter 5% CO₂ wurde die Zelldichte bestimmt. Mittelwerte der Zelldichte wurden aus 3 individuellen Löchern einer PNA Konzentration ermittelt. Es zeigte sich, dass PNA-13 die Proliferation der REH Zellen hemmt. Nach > 4 Tagen Inkubationszeit ist die Hemmung durch PNA-6 stärker als durch ein entsprechendes Phosphorothioat-Oligonucleotid.

### Beispiel 11: Synthese von Aminohexyl-p-spacer18-p-t(oeg) at tcc gtc at-aminohexyl-p (PNA-7)

Die Herstellung erfolgt analog wie in Beispiel 2 beschrieben in einer 1 µmol Synthese. Nach Aufbau des Carboxyterminus und Synthese des PNA-Teils wird im letzten Zyklus allerdings ein auf Hydroxyethylglycin basierender Baustein mit Thymin als Nucleobase (oegT) gekuppelt. Nach Spaltung der Dmt-Gruppe wird die freie Hydroxyfunktion mit dem Spacer18 Phsophoramidit (Figur 4c) und nach nochmaliger Detritylierung mit dem Aminomodifier-C6 Phosphoramidit 13 (Figur 4d) unter Tetrazol-Katalyse gekuppelt und anschliessend mit Jodwasser oxidiert. Durch Behandlung mit konz. Ammoniak bei 50°C spaltet man das Oligomer vom Träger und entfernt gleichzeitig alle basenlabilen Schutzgruppen. Dann wird die terminale Mmt-Schutzgruppe durch Behandlung mit 80% Essigsäure entfernt. Man erhält 57 OD des Rohproduktes, das durch GelElektrophorese gereinigt wurde. Man erhält 7.4 OD Produkt, das im Massenspektrum das erwartete Molekulargewicht 3647.5 (ber. 3648.5) zeigt.

### Abkürzungsverzeichnis:

| | |
|---|---|
| ACN | Acetonitril |
| BOC. | tert-Butyloxycarbonyl |
| C, c | pseudo-iso-Cytosin |
| COS | CV1 Origin SV 40 |
| CPG | controlled pore glass |
| DCM | Dichlormethan |
| DIPEA | Diisopropylethylamin |
| DMEM | Dulbecco's MEM |
| DMF | Dimethylformamid |
| Dmt | Dimethoxytrityl |
| DNA | Desoxyribonukleinsäure |
| DNP | Dinitroaryl |
| FITC | Fluoresceinisothiocyanat |
| Fmoc | Fluorenylmethoxycarbonyl |
| HATU | O-(7-Azabenzotriazol-1-yl)-1,1,3,3-tetramethyluronium-hexafluorphosphat |
| HBTU | O-(Benzotriazol-1-yl)-1,1,3,3-tetramethyluronium-hexafluorphosphat |
| hex | -NH-(CH₂)₆-OH |
| MEM | Modified Eagle's minimal essential medium |
| Mmt | Monomethoxytrityl |
| OD | Optische Dichte |
| oeg | N-(2-Hydroxyethyl)glycin |
| PAA | Polyacrylamid |
| PG | Schutzgruppe |
| PNA | Polyamidnukleinsäure |
| RNA | Ribonukleinsäure |
| TBTU | O-(Benzotriazol-1-yl)-1,1,3,3-tetramethyluronium-tetrafluoroborat |
| TCA | Trichloressigsäure |
| THF | Tetrahydrofuran |
| TR | säurelabile Schutzgruppe |

Figuren 1a, 1 b, 2b und 3b zeigen Beispiele für endständige Reste Z und Z'.

Figuren 2a und 3a zeigen Beispiele für verbrückende Reste X und X'.

Figuren 4a, 4b, 4c und 4d zeigen Beispiele für Phosphorylierungsreagenzien.

Figuren 5a und 5b zeigen Beispiele für die einfache (A, B) und multiple (C bis E) Derivatisierung am N-Terminus von PNA.

Figur 6 zeigt Beispiele für Träger-gebundene Reagenzien für die Festphasensynthese.

Figuren 7, 8 und 9 zeigen Beispiele für die Synthese C- und N-terminal modifizierter PNA.

## Patentansprüche

1. PNA-Derivat der Formel I 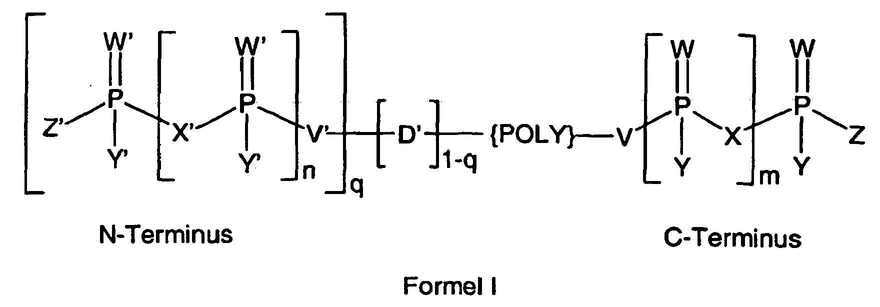 wobei
q gleich 0 oder 1 ist,
D' gleich Hydroxy, Mercapto, Amino, Alkylamino oder Acylamino ist,
V unabhängig voneinander gleich Sauerstoff, Schwefel, NR₁ ist,
V' unabhängig voneinander gleich Sauerstoff, Schwefel, NR₁, eine Gruppe U-(CR₃R₄)_{u'}-C(O)-NH oder eine Gruppe U-(CH₂CH₂O)_{u'-}CH₂-C(O)-NH ist,
U unabhängig voneinander gleich Sauerstoff, Schwefel oder NH ist,
u' unabhängig voneinander gleich 1 bis 10 ist, vorzugsweise 1 bis 4, besonders bevorzugt 1,
W und W' unabhängig voneinander gleich Sauerstoff, Schwefel oder NR₁ sind,
Y und Y' unabhängig voneinander gleich Hydroxy, Mercapto, Oxyanion, Thioat oder NR₁ R₂ sind,
X und X' unabhängig voneinander gleich eine Gruppe U-(C₂-C₂₂₋Alkandiyl)-U oder eine Gruppe U-(CH₂CH₂-O)_{u'} ist, oder gleich eine Markergruppe, oder eine Gruppe zur Quervernetzung, oder eine Gruppe, die die intrazelluläre Aufnahme begünstigt, oder eine Gruppe, die die Bindungsaffinität des PNA-Derivats an Nukleinsäuren steigert, ist, beispielsweise ein bifunktioneller Fluorescein-, Rhodamin-, TAMRA-, Biotin-, Pyren-, Dinitrophenyl-, Cholesteryl-, Acridin-, Adamantyl-, Vitamin E-, Cyaninfarbstoff-, Dabcyl-, Edans-, Lexitropsin-, Psoralen-, BODIPY-, ROX-, R6G- oder Digoxigenin-Rest,
Z und Z' unabhängig voneinander gleich Hydroxy, Mercapto, Oxyanion, Thioat oder NR₁ R₂, C₁-C₂₂-Alkyl, C₁-C₈-Arylalkyl, C₁-C₂₂₋Alkyl-U, C₁-C₈-Arylalkyl-U, Hydroxy-C₁-C₁₈-U, Aminoalkyl-U, Mercaptoalkyl-U ist,
oder eine Gruppe der Formel R₇(CH₂CH₂-O)ₘ ist, wobei R₇ gleich Hydroxy, Amino oder C₁-C₂₂-Alkoxy und m gleich 1 bis 100 ist, vorzugsweise 2 bis 10,
oder gleich eine Markergruppe, oder eine Gruppe zur Quervernetzung, oder eine Gruppe, die die intrazelluläre Aufnahme begünstigt, oder eine Gruppe, die die Bindungs-affinität des PNA-Derivats an Nukleinsäuren steigert, ist, beispielsweise ein monofunktioneller oder bifunktioneller Fluorescein-, Rhodamin-, TAMRA-, Biotin-, Pyren-, Dinitrophenyl-, Cholesteryl-, Acridin-, Adamantyl-, Vitamin E-, Cyaninfarbstoff-, Dabcyl-, Edans-, Lexitropsin-, Psoralen-, BODIPY-, ROX-, R6G- oder Digoxigenin-Rest,
R₁ und R₂ unabhängig voneinander ein Rest bestehend aus Wasserstoff oder C₁-C₆-Alkyl bedeuten, vorzugsweise Wasserstoff,
R₃ und R₄ unabhängig voneinander ein Rest bestehend aus Wasserstoff oder C₁-C₆-Alkyl oder den Rest einer Aminosäure-Seitenkette bedeuten, vorzugsweise Wasserstoff, wobei benachbarte Reste R₃ und R₄ in V' auch einen C₅-C₈-Cycloalkyl-Ring bilden können,
n gleich 0 bis 10 ist, vorzugsweise 0 bis 3,
m gleich 0 bis 10 ist, vorzugsweise 0 bis 3,
und wobei {POLY} beschrieben durch die Formel II wird, 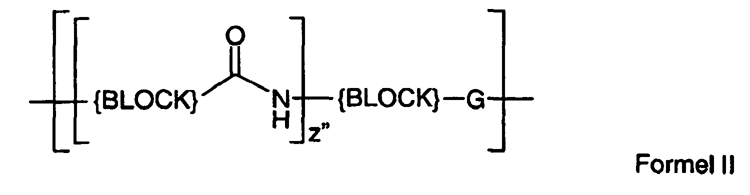 wobei ferner {BLOCK} unabhängig voneinander eine Gruppe ist ausgewählt aus Formel IIIA, 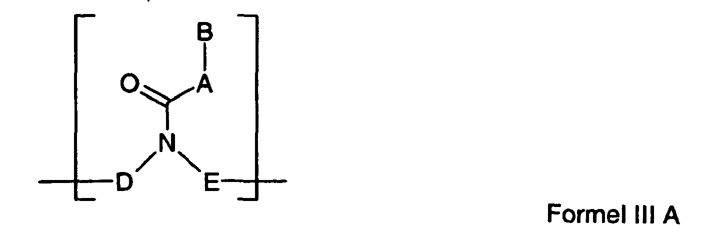 oder aus Formel IIIB,
G ausgewählt wird aus den Gruppen (CR₅R₆)_{u'}, C(O)NH-(CR₁R₂)_{t'} oder C(O)NH-(CH₂CH₂O)_{u'}-CH₂CH₂ ist, wobei t' gleich 2 bis 10 ist, vorzugsweise 6,
A unabhängig voneinander eine Gruppe (CR₁R₂)ₛ ist, wobei s gleich 1 bis 3 ist, vorzugsweise 1,
B unabhängig voneinander entweder ein aromatischer Rest, der auch heteroaromatischen Charakter besitzen kann, oder Wasserstoff, oder Hydroxy oder C₁-C₁₈-Alkyl ist, oder eine in der Nucleotidchemie übliche natürlich vorkommende oder eine nicht natürlich vorkommende Nucleobase oder deren Prodrugform ist,
D unabhängig voneinander eine Gruppe (CR₃R₄)ₜ ist, wobei t gleich 2 bis 10 ist, vorzugsweise 2 bis 4, besonders bevorzugt 2,
E unabhängig voneinander eine Gruppe (CR₅R₆)_{u'} ist, wobei benachbarte Reste R₅ und R₆ auch einen C₅-C₈-Cycloalkyl-Ring oder eine Spiroverbindung bilden können,
R₅ und R₆ unabhängig voneinander einen Rest bestehend aus Wasserstoff oder C₁-C₆-Alkyl oder den Rest einer Aminosäure-Seitenkette bedeuten, vorzugsweise Wasserstoff,
und wobei u', R₁, R₂, R₃ und R₄ die gleiche Bedeutung wie oben beschrieben haben,
sowie physiologisch verträglichen Salze des PNA-Derivates der Formel I, 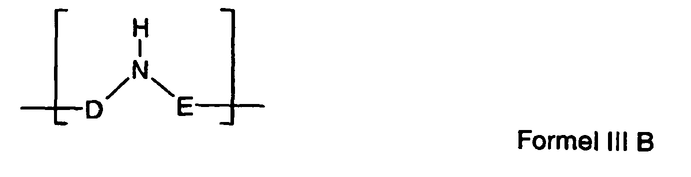 oder aus den Formeln IV A bis IV G, 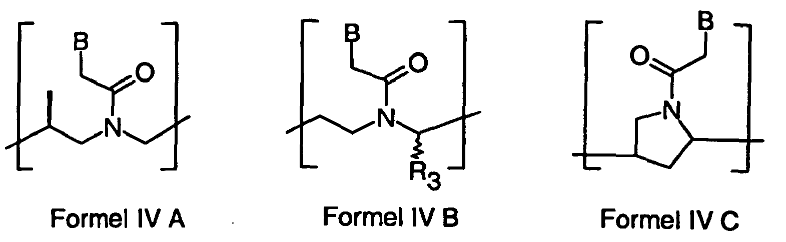 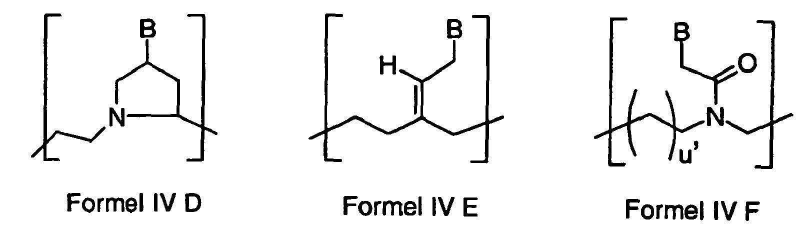 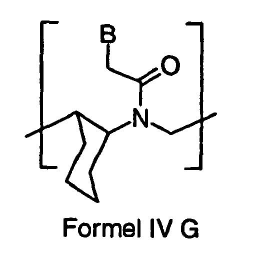 wobei jeder Baustein {BLOCK} verschieden sein kann,
und ferner gilt, dass
z" gleich 0 bis 100 ist, vorzugsweise 1-20, besonders bevorzugt 4-15,
mit den Massgaben, dass mindestens ein Rest Y, Y', Z oder Z' gleich Hydroxy, Mercapto, Oxyanion oder Thioat ist, und dass mindestens ein Rest B eine Nucleobase ist.

2. PNA-Derivat nach Anspruch 1, wobei mindestens ein Rest Y, Y', Z oder Z' in Formel I in einem pH-Bereich von 4,5 bis 14, vorzugsweise 6,5 bis 12, besonders bevorzugt 6,5 bis 9 gleich Oxyanion oder Thioat ist.

3. PNA-Derivat nach einem der Ansprüche 1 bis 2, wobei n und m unabhängig voneinander gleich 0 sind.

4. PNA-Derivat nach einem der Ansprüche 1 bis 3, wobei q gleich 1 ist.

5. PNA-Derivat nach einem der Ansprüche 1 bis 4, wobei W und W' gleich Oxo sind.

6. PNA-Derivat nach einem der Ansprüche 1 bis 5, wobei Y und Y' gleich Hydroxy oder Oxyanion sind.

7. PNA-Derivat nach einem der Ansprüche 1 bis 6, wobei V und V' gleich Oxy sind.

8. PNA-Derivat nach einem der Ansprüche 1 bis 7, wobei X und X' unabhängig voneinander gleich eine Gruppe U-(C₂-C₂₂-Alkandiyl)-U, bevorzugt O-(C₂₋C₂₂-Alkandiyl)-O, besonders bevorzugt O-(CH₂)₂₋₆O sind, oder gleich eine Gruppe U-(CH₂CH₂-O)_{u'}, bevorzugt O(CH₂CH₂-O)_{u'}-, wobei u' 1 bis 6 ist.

9. PNA-Derivat nach einem der Ansprüche 1 bis 8, wobei X, X', Z und Z' unabhängig voneinander ausgewählt werden aus der Gruppe Fluorescein, Rhodamin, TAMRA oder Cyanin-Farbstoff, Biotin, Dabcyl, Psoralen, Acridin, DNP, Cholesterol, Vitamin E-, Dabcyl-, Edans-, Lexitropsin-, Psoralen-, BODIPY-, ROX-, R6G- oder Digoxigenin.

10. PNA-Derivat nach einem der Ansprüche 1 bis 9, wobei X, X', Z und Z' unabhängig voneinander ausgewählt werden aus der Gruppe Monophosphat, Biotin-Derivat und Fluoreszein-Derivat.

11. PNA-Derivat nach einem der Ansprüche 1 bis 9, wobei Z ein Fluoreszenz-Marker ist und Z' ein Quencher.

12. PNA-Derivat nach einem der Ansprüche 1 bis 9, wobei Z ein Quencher ist und Z' ein Fluoreszenz-Marker.

13. PNA-Derivat nach einem der Ansprüche 1 bis 9, wobei Z und Z' unabhängig voneinander ein C₁-C₂₂-Alkyl-Rest sind,
oder ein C₁-C₂₂-U-Rest, bevorzugt ein C₁-C₂₂-Alkoxy-Rest, besonders bevorzugt C₁₆-Alkoxy,
oder Hydroxy-C₁-C₁₈-U, bevorzugt Hydroxy-C₁-C₁₈-O, besonders bevorzugt HO-(CH₂)₃₋₁₂O,
oder ein Aminoalkyl-U-Rest, bevorzugt ein Aminoalkoxy-Rest, besonders bevorzugt 6-Aminohexoxy oder 5-Aminopentoxy,
oder eine Gruppe der Formel R₇-(CH₂CH₂-O)ₘ ist, wobei R₇ bevorzugt OH oder NH₂ ist und m gleich 1 bis 6 ist, besonders bevorzugt HO(CH₂CH₂-O)₂, HO(CH₂CH₂-O)₆ und H₂N-(CH₂CH₂-O)₂,
oder ein Mercaptoalkyl-V-Rest, bevorzugt ein Mercaptoalkoxy-Rest, besonders bevorzugt 6-Mercaptohexyloxy.

14. PNA-Derivat nach einem der Ansprüche 1 bis 3, wobei q gleich 0 ist.

15. PNA-Derivat nach Anspruch 14, wobei D' gleich Acylamino ist, vorzugsweise Acetylamino.

16. PNA-Derivat nach einem der Ansprüche 1 bis 15, wobei D gleich (CH₂)ₜ, bevorzugt (CH₂)₂ ist.

17. PNA-Derivat nach einem der Ansprüche 1 bis 16, wobei A, E und G gleich CH₂ sind.

18. PNA-Derivat nach einem der Ansprüche 1 bis 17, wobei B gleich Adenin, Cytosin, 5-Methylcytosin, Guanin, Thymin und Uracil, oder gleich Purin, 2,6-Diaminopurin, N⁴N⁴-Ethanocytosin, N⁶N⁶-Ethano-2,6-diaminopurin, 5-(C₃₋C₆)-Alkinyl-uracil, 5-(C₃-C₆)-Alkinyl-cytosin, 5-(1-Propargylamino)-uracil, 5-(1-Propargylamino)-cytosin, Phenoxazin, 9-Aminoethoxyphenoxazin, 5-Fluor-uracil oder Pseudoisocytosin, 5-(Hydroxymethyl)uracil, 5-Aminouracil, Pseudouracil, Dihydrouracil, 5-(C₁-C₆)-Alkyl-uracil, 5-(C₁-C₆)-Alkyl-cytosin, 5-(C₂-C₆)-Alkenyl-cytosin, 5-Fluorcytosin, 5-Chloruracil, 5-Chlorcytosin, 5-Bromuracil, 5-Bromcytosin, 7-Deazaadenin, 7-Deazaguanin, 8-Azapurin, oder ein 7-Deaza-7-substituiertes Purin ist.

19. PNA-Derivat nach einem der Ansprüche 1 bis 18, wobei die Basensequenz gegen Teile von Tumorsupressor-Genen, Onkogenen oder Telomerasen oder deren Transkriptions-Produkte gerichtet ist.

20. PNA-Derivat nach Anspruch 19, wobei die Basensequenz des PNA-Teils gegen den Translationsstart von HA-ras mRNA gerichtet ist.

21. PNA-Derivat nach einem der Ansprüche 1-10 oder 13-20 zur Verwendung als Arzneimittel.

22. Verwendung eines PNA-Derivates nach einem der Ansprüche 1-10 oder 15-22 zur Herstellung eines Arzneimittels für die Tumortherapie.

23. PNA-Derivat nach einem der Ansprüche 1 bis 20 zur Verwendung als Diagnostikum.

24. Verwendung eines PNA-Derivats nach einem der Ansprüche 1 bis 20 zum Nachweis von Mikroorganismen und/oder Viren.

25. Verwendung eines PNA-Derivats nach einem der Ansprüche 1 bis 20 zum Nachweis und/oder Quantifizierung von Nukleinsäuren.

26. Verwendung eines PNA-Derivats nach einem der Ansprüche 1 bis 20 als Nachweisreagenz für die in-situ- oder Fluoreszenz-in-situ-Hybridisierung.

27. Verwendung eines PNA-Derivats nach einem der Ansprüche 1, bis 20 als Antisense-, Anti-Gen-, Decoy-, oder Chimeraplasty-Agenz.

28. Verwendung eines PNA-Derivates nach einem der Ansprüche 11 oder 12 als Molecular Beacon.

29. Nachweisreagenz enthaltend ein PNA-Derivat nach einem der Ansprüche 1 bis 20.

30. PNA-Chip, enthaltend ein PNA-Derivat nach einem der Ansprüche 1 bis 20.

31. Biosensor enthaltend ein PNA-Derivat nach einem der Ansprüche 1 bis 20.

32. Arzneimittel, enthaltend ein PNA-Derivat nach einem der Ansprüche 1-10 oder 15-22 und gegebenenfalls weitere pharmakologisch verträgliche Zusatz-und/oder Trägerstoffe.

33. Antisense-, Anti-Gen-, Decoy-, oder Chimeraplasty-Agenz, enthaltend ein PNA-Derivat nach einem der Ansprüche 1 bis 20.

34. Verfahren zur Herstellung eines PNA-Derivats der Formel I, wobei
a) der C-Terminus einer Amidnukleinsäure mit einem festphasengebundenen Phosphorylierungs-Reagenz verknüpft wird, oder eine mit C-terminal phosphorylierte Amidnukleinsäure an einen festen Träger gebunden wird,
b) das Rückgrat des PNA-Oligomers durch sequentiell durch Kupplung mit Amidonukleinsäure-Monomeren verlängert wird,
c) gegebenfalls am N-Terminus mit einem Phosphorylierungsreagenz umgesetzt wird.

35. Verfahren nach Anspruch 34, wobei die PNA unter Verwendung der Schutzgruppen t-Butyloxycarbonyl (BOC), 9-Fluorenylmethoxycarbonyl (Fmoc) oder Monomethoxytrityl (Mmt) hergestellt wird.

36. Verfahren nach einem der Ansprüche 34 oder 35, wobei die PNA unter Verwendung von festen Trägern hergestellt wird.

37. Verfahren nach Anspruch 36, wobei als fester Träger CPG^{®}, Tentagel^{®} oder Aminomethylpolystyren verwendet wird.

38. Verfahren zur Herstellung eines Arzneimittels, wobei
a) ein PNA-Derivat nach einem der Ansprüche 1-10 oder 15-20 hergestellt wird, und
b) gegebenfalls mit weiteren pharmakologisch verträglichen Zusatz-und/oder Trägerstoffen versetzt wird.

39. Verfahren zur Herstellung eines PNA-Chips, wobei ein PNA-Derivat nach einem der Ansprüche 1 bis 20 entweder zuerst hergestellt und dann auf einem festen Träger fixiert wird, oder das PNA-Derivat direkt auf dem Träger hergestellt wird.

40. Verfahren zur Herstellung eines PNA-Derivats der Formel I gemäß Anspruch 34 bis 37, ferner **dadurch gekennzeichnet, dass** die PNA unter Ausnutzung des sauren Charakters des Phosporrestes mittels Chromatographie oder Elektrophorese aufgereinigt wird.

41. Verfahren nach Anspruch 40, wobei das PNA-Derivat durch Chromatographie mittels einer basischen stationären Phase und eines Gradienten eines sauren oder salzhaltigen Eluent gereinigt werden.

42. Verfahren nach Anspruch 41, wobei die stationäre Phase ein Anionenaustauscher oder eine Mixed-Mode-Phase ist.

## Claims

1. A PNA derivative of the formula I 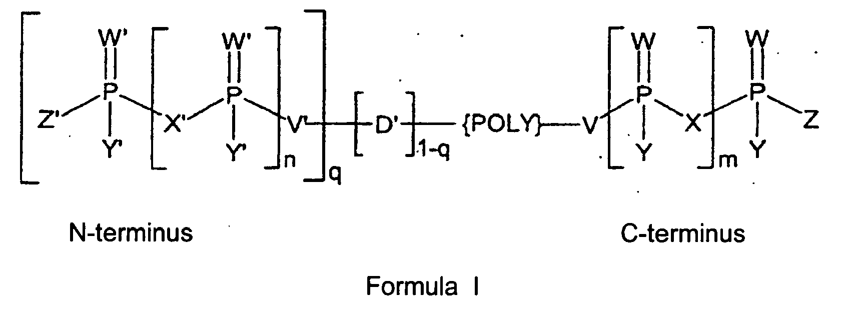 where
q is 0 or 1,
D' is hydroxyl, mercapto, amino, alkylamino or acylamino,
V is, independently of each other, oxygen, sulfur, NR₁,
V' is, independently of each other, oxygen, sulfur, NR₁, a group U-(CR₃R₄)ᵤ'-C(O)-NH or a group U-(CH₂CH₂O)ᵤ'-CH₂-C(O)-NH,
U is, independently of each other, oxygen, sulfur or NH,
u' is, independently of each other, from 1 to 10, preferably from 1 to 4, particularly preferably 1,
W and W' are, independently of each other, oxygen, sulfur or NR₁,
Y and Y' are, independently of each other, hydroxyl, mercapto, oxyanion, thioate or NR₁R₂,
X and X' are, independently of each other, a group U-(C₂-C₂₂-alkanediyl)-U or a group U-(CH₂CH₂-O)ᵤ',
or are a labeling group or a group for crosslinking, or a group which promotes intracellular uptake, or a group which increases the binding affinity of the PNA derivative for nucleic acids, for example a bifunctional fluorescein, rhodamine, TAMRA, biotin, pyrene, dinitrophenyl, cholesteryl, acridine, adamantyl, vitamin E, cyanine dye, Dabcyl, Edans, lexitropsin, psoralen, BODIPY, ROX, R6G or digoxigenin radical,
Z and Z' are, independently of each other, hydroxyl, mercapto, oxyanion, thioate or NR₁ R₂, C₁-C₂₂-alkyl, C₁-C₈-arylalkyl, C₁-C₂₂-alkyl-U, C₁-C₈₋arylalkyl-U, hydroxy-C₁-C₁₈-U, aminoalkyl-U, mercaptoalkyl-U, or a group of the formula R₇(CH₂CH₂-O)ₘ, where R₇ is hydroxyl, amino or C₁-C₂₂-alkoxy, and m is from 1 to 100, preferably from 2 to 10, or are a labeling group, or a group for crosslinking, or a group which promotes intracellular uptake, or a group which increases the binding affinity of the PNA derivative for nucleic acids, for example a monofunctional or bifunctional fluorescein, rhodamine, TAMRA, biotin, pyrene, dinitrophenyl, cholesteryl, acridine, adamantyl, vitamin E, cyanine dye, Dabcyl, Edans, lexitropsin, psoralen, BODIPY, ROX, R6G or digoxigenin radical,
R₁ and R₂ are, independently of each other, a radical consisting of hydrogen or C₁-C₆-alkyl, preferably hydrogen,
R₃ and R₄ are, independently of each other, a radical consisting of hydrogen or C₁-C₆-alkyl, or the radical of an amino acid side chain, preferably hydrogen, it being possible for adjacent radicals R₃ and R₄ in V' to also form a C₅-C₈-cycloalkyl ring,
n is from 0 to 10, preferably from 0 to 3,
m is from 0 to 10, preferably from 0 to 3,
and where {POLY} is described by the formula II 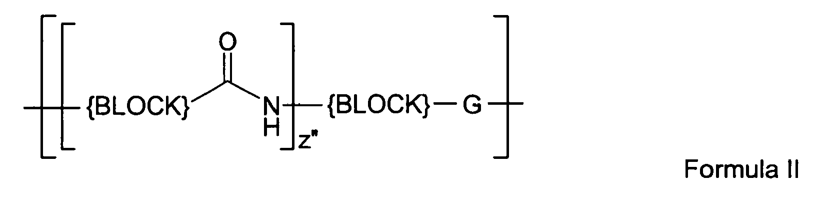 where {BLOCK} is also, independently of each other, a group selected from formula IIIA, 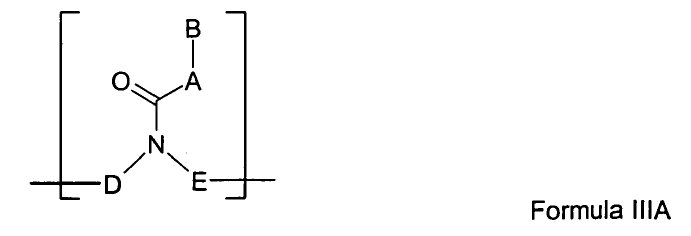 or from formula IIIB, 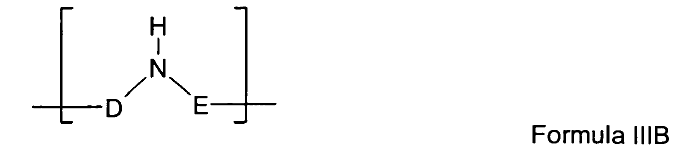 or from the formulae IV A to IV G, 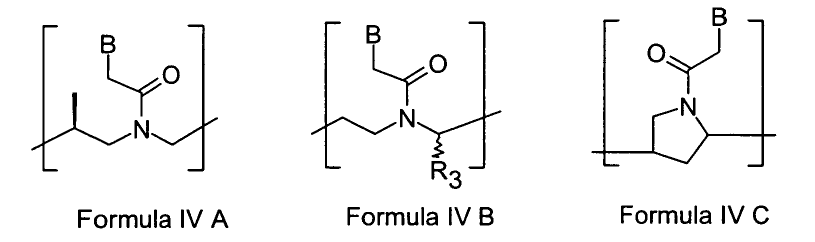 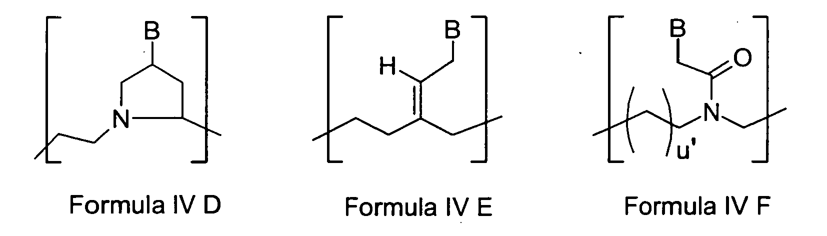 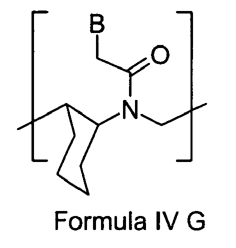 where each {BLOCK} building block can be different,
and it furthermore applies that
z" is from 0 to 100, preferably 1-20, particularly preferably 4-15,
G is selected from the groups (CR₅R₆)_{u'}, C(O)NH-(CR₁R₂)_{t'} or C(O)NH-(CH₂CH₂O)_{u'}-CH₂CH₂, where t' is from 2 to 10, preferably 6,
A is, independently of each other, a group (CR₁ R₂)ₛ, where s is from 1 to 3, preferably 1,
B is, independently of each other, either an aromatic radical, which can also possess heteroaromatic character, or hydrogen, or hydroxyl or C₁-C₁₈-alkyl,
or a nucleobase which occurs naturally, and is customary in nucleotide chemistry, or which does not occur naturally, or its prodrug form,
D is, independently of each other, a group (CR₃R₄)ₜ, where t is from 2 to 10, preferably from 2 to 4, particularly preferably 2,
E is, independently of each other, a group (CR₅R₆)_{u'}, where adjacent R₅ and R₆ radicals can also form a C₅-C₈-cycloalkyl ring or a spiro compound,
R₅ and R₆ are, independently of each other, a radical consisting of hydrogen or C₁-C₆-alkyl or the radical of an amino acid side chain, preferably hydrogen,
And where u', R₁, R₂, R₃, and R₄, have the same meaning as described above,
as well as physiologically tolerated salts of the PNA derivative of the formula I,
with the provisos that at least one Y, Y', Z or Z' radical is hydroxyl, mercapto, oxyanion or thioate, and that at least one B radical is a nucleobase.

2. A PNA derivative as claimed in claim 2, wherein at least one Y, Y', Z or Z' radical in formula I is oxyanion or thioate in a pH range from 4.5 to 14, preferably from 6.5 to 12, particularly preferably from 6.5 to 9.

3. A PNA derivative as claimed in either claims 1 and 2, wherein n and m are, independently of each other, 0.

4. A PNA derivative as claimed in one of claims 1 to 3, wherein q is 1.

5. A PNA derivative as claimed in one of claims 1 to 4, wherein W and W' are oxo.

6. A PNA derivative as claimed in one of claims 1 to 5, wherein Y and Y' are hydroxyl or oxyanion.

7. A PNA derivative as claimed in one of claims 1 to 6, wherein V and V' are oxy.

8. A PNA derivative as claimed in one of claims 1 to 7, wherein X and X' are, independently of each other, a group U-(C₂-C₂₂-alkanediyl)-U, preferably O-(C₂-C₂₂-alkanediyl)-O, particularly preferably O-(CH₂)₂₋₆O, or are a group U-(CH₂CH₂-O)_{u'}, preferably O(CH₂CH₂-O)_{u'}, where u' is from 1 to 6.

9. A PNA derivative as claimed in one of claims 1 to 8, wherein X, X', Z and Z' are selected, independently of each other, from the group fluorescein, rhodamine, TAMRA or cyanine dye, biotin, dabcyl, psoralen, acridine, DNP, cholesterol, vitamin E, dabcyl, edans, lexitropsin, psoralen, BODIPY, ROX, R6G or digoxigenin.

10. A PNA derivative as claimed in one of claims 1 to 9, wherein X, X', Z and Z' are selected, independently of each other, from the group monophosphate, biotin derivative and fluorescein derivative.

11. A PNA derivative as claimed in one of claims 1 to 9, wherein Z is a fluorescence label and Z' is a quencher.

12. A PNA derivative as claimed in one of claims 1 to 9, wherein Z is a quencher and Z' is a fluorescence label.

13. A PNA derivative as claimed in one of claims 1 to 9, wherein Z and Z' are, independently of each other, a C₁-C₂₂-alkyl radical,
or a C₁-C₂₂-U radical, preferably a C₁-C₂₂-alkoxy radical, particularly preferably C₁₆-alkoxy,
or hydroxy-C₁-C₁₈-U, preferably hydroxy-C₁-C₁₈-O, particularly preferably HO-(CH₂)₃₋₁₂O,
or an aminoalkyl-U radical, preferably an aminoalkoxy radical, particularly preferably 6-aminohexoxy or 5-aminopentoxy,
or a group of the formula R₇-(CH₂CH₂-O)ₘ, where R₇ is preferably OH or NH₂ and m is from 1 to 6, particularly preferably HO(CH₂CH₂-O)₂,
HO(CH₂CH₂-O)₆ and H₂N-(CH₂CH₂-O)₂, or a mercaptoalkyl-V radical, preferably a mercaptoalkoxy radical, particularly preferably 6-mercaptohexyloxy.

14. A PNA derivative as claimed in one of claims 1 to 3, wherein q is 0.

15. A PNA derivative as claimed in claim 14, wherein D' is acylamino, preferably acetylamino.

16. A PNA derivative as claimed in one of claims 1 to 15, wherein D is (CH₂)ₜ, preferably (CH₂)₂.

17. A PNA derivative as claimed in one of claims 1 to 6, wherein A, E and G are CH₂.

18. A PNA derivative as claimed in one of claims 1 to 7, wherein B is adenine, cytosine, 5-methylcytosine, guanine, thymine and uracil, or is purine, 2,6-diaminopurine, N⁴N⁴-ethanocytosine, N⁶N⁶-ethano-2,6-diaminopurine, 5-(C₃-C₆)-alkynyluracil, 5-(C₃-C₆)-alkynyl-cytosine, 5-(1-propargylamino)uracil, 5-(1-propargylamino)cytosine, phenoxazine, 9-aminoethoxyphenoxazine, 5-fluorouracil or pseudoisocytosine, 5-(hydroxymethyl)uracil, 5-aminouracil, pseudouracil, dihydrouracil, 5-(C₁₋C₆)-alkyluracil, 5-(C₁-C₆)-alkyl-cytosine, 5-(C₂-C₆)-alkenylcytosine, 5-fluorocytosine, 5-chlorouracil, 5-chlorocytosine, 5-bromouracil, 5-bromocytosine, 7-deazaadenine, 7-deazaguanine, 8-azapurine, or a 7-deaza-7-substituted purine.

19. A PNA derivative as claimed in one of claims 1 to 18, wherein the base sequence is directed against parts of tumor suppressor genes, oncogenes or telomerases, or their transcription products.

20. A PNA derivative as claimed in claim 19, wherein the base sequence of the PNA moiety is directed against the translation start of HA-ras mRNA.

21. A PNA derivative as claimed in one of claims 1-10 or 13-20 for use as a pharmaceutical.

22. The use of a PNA derivative as claimed in one of claims 1-10 or 15-22 for producing a pharmaceutical for tumor therapy.

23. A PNA derivative as claimed in one of claims 1 to 20 for use as a diagnostic agent.

24. The use of a PNA derivative as claimed in one of claims 1 to 20 for detecting microorganisms and/or viruses.

25. The use of a PNA derivative as claimed in one of claims 1 to 20 for detecting and/or quantifying nucleic acids.

26. The use of a PNA derivative as claimed in one of claims 1 to 20 as a detection reagent for in-situ hybridization or fluorescence in-situ hybridization.

27. The use of a PNA derivative as claimed in one of claims 1 to 20 as an antisense agent, anti-gene agent, decoy agent or chimeraplasty agent.

28. The use of a PNA derivative as claimed in one of claims 11 or 12 as a molecular beacon.

29. A detection reagent, comprising a PNA derivative as claimed in one of claims 1 to 20.

30. A PNA chip, comprising a PNA derivative as claimed in one of claims 1 to 20.

31. A biosensor, comprising a PNA derivative as claimed in one of claims 1 to 20.

32. A pharmaceutical, comprising a PNA derivative as claimed in one of claims 1-10 or 15-22 and, where appropriate, other pharmacologically tolerated additives and/or excipients.

33. An antisense agent, anti-gene agent, decoy agent or chimeraplasty agent, comprising a PNA derivative as claimed in one of claims 1 to 20.

34. A process for preparing a PNA derivative of the formula I, wherein
a) the C terminus of an amidonucleic acid is linked to a solid phase-bound phosphorylating reagent, or an amidonucleic acid which is phosphorylated C-terminally is bound to a solid support,
b) the backbone of the PNA oligomer is extended sequentially by coupling with amidonucleic acid monomers,
c) if desired, the N-terminus is reacted with a phosphorylating reagent.

35. The process as claimed in claim 34, wherein the PNA is prepared using the t-butyloxycarbonyl (BOC), 9-fluorenylmethoxycarbonyl (Fmoc) or monomethoxytrityl (Mmt) protecting groups.

36. The process as claimed in one of claims 34 and 35, wherein the PNA is prepared using solid supports.

37. The process as claimed in claim 38, wherein CPG^{®}, tentagel^{®} or aminomethylpolystyrene is used as the solid support.

38. A process for producing a pharmaceutical, wherein
a) a PNA derivative as claimed in one of claims 1-10 or 15-20 is prepared, and
b) further pharmacologically tolerated additives and/or excipients are added to it, where appropriate.

39. A process for producing a PNA chip, wherein a PNA derivative as claimed in one of claims 1 to 20 is either firstly prepared and then fixed onto a solid support, or the PNA derivative is prepared directly on the support.

40. A process for preparing a PNA derivative of the formula I as claimed in claims 34 to 37, wherein, in addition, the PNA is purified by means of chromatography or electrophoresis while exploiting the acid character of the phosphorus radical.

41. The process as claimed in claim 40, wherein the PNA derivative is purified by means of chromatography using a basic stationary phase and a gradient of an acid or salt-containing eluent.

42. The process as claimed in claim 41, wherein the stationary phase is an anion exchanger or a mixed-mode phase.

## Revendications

1. Dérivé de PNA de formule I : 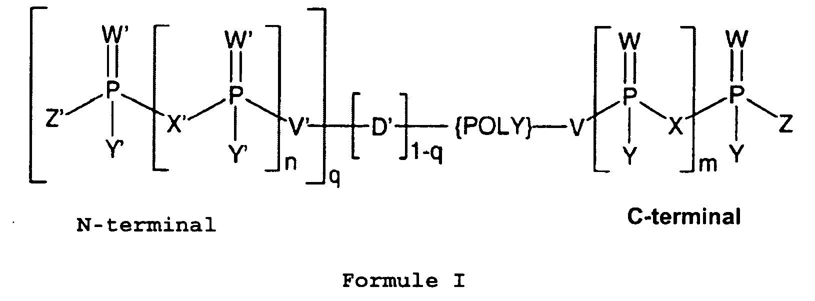 où
q est égal à 0 ou à 1,
D' est un groupement hydroxy, mercapto, amino, alkylamino ou acylamino,
les groupements V sont, indépendamment l'un de l'autre, de l'oxygène, du soufre ou NR₁,
les groupements V' sont, indépendamment l'un de l'autre, de l'oxygène, du soufre ou NR₁, un groupement U-(CR₃R₄)_{u'}-C(O)-NH ou un groupement U-(CH₂CH₂O)_{u'}-CH₂₋C(O)-NH,
les groupements U représentent, indépendamment l'un de l'autre, de l'oxygène, du soufre ou NH,
les indices u' ont, indépendamment l'un de l'autre, une valeur de 1 à 10, de préférence de 1 à 4, mieux encore de 1,
les groupements W et W' sont, indépendamment l'un de l'autre, de l'oxygène, du soufre ou NR₁,
les groupements T et Y' représentent, indépendamment l'un de l'autre, un groupement hydroxy, mercapto, oxyanion, thioate ou NR₁R₂,
les groupements X et X' sont, indépendamment l'un de l'autre, un groupement U- (alcanediyle en C₂-C₂₂) -U ou un groupement U-(CH₂CH₂-O)_{u'}, ou un groupement marqueur, ou un groupement de réticulation transversale, ou un groupement qui favorise l'absorption intracellulaire, ou un groupement qui augmente l'affinité de liaison du dérivé de PNA avec les acides nucléiques, par exemple un radical bifonctionnel fluorescéine, rhodamine, TAMRA, biotine, pyrène, dinitrophényle, cholestéryle, acridine, adamantyle, vitamine E, colorant de cyanine, dabcyle, edans, lexitropsine, psoralène, BODIPY, ROX, R6G ou digoxygénine,
les groupements Z et Z' sont, indépendamment l'un de l'autre, un groupement hydroxy, mercapto, oxyanion, thioate ou NR₁R₂, alkyle en C₁-C₂₂, arylalkyle en C₁-C₈, alkyle en C₁-C₂₂-U, arylalkyle en C₁-C₈-U, hydroxyalkyle en C₁-C₁₈-U, aminoalkyle-U, mercaptoalkyle-U,
ou sont un groupement de formule R₇(CH₂CH₂-O)ₘ, dans laquelle R₇ représente un groupement hydroxy, amino ou alcoxy en C₁-C₂₂ et m vaut 1 à 100, de préférence 2 à 10,
ou sont un groupement marqueur, ou un groupement de réticulation transversale, ou un groupement qui favorise l'absorption intracellulaire, ou un groupement qui augmente l'affinité de liaison du dérivé de PNA avec les acides nucléiques, par exemple un radical monofonctionnel ou bifonctionnel fluorescéine, rhodamine, TAMRA, biotine, pyrène, dinitrophényle, cholestéryle, acridine, adamantyle, vitamine E, colorant de cyanine, dabcyle, edans, lexitropsine, psoralène, BODIPY, ROX, R6G ou digoxygénine,
les groupements R₁ et R₂ signifient, indépendamment l'un de l'autre, un radical constitué de l'hydrogène ou d'un alkyle en C₁-C₆, de préférence l'hydrogène,
les groupements R₃ et R₄ représentent, indépendamment l'un de l'autre, un radical constitué de l'hydrogène ou d'un alkyle en C₁-C₆ ou le radical d'une chaîne latérale d'acide aminé, de préférence l'hydrogène, des radicaux adjacents R₃ et R₄ de V' pouvant également former un cycle cycloalkyle en C₅-C₈,
n est égal à 0 à 10, de préférence à 0 à 3,
m est égal à 0 à 10, de préférence à 0 à 3, et où {POLY} est défini par la formule II : 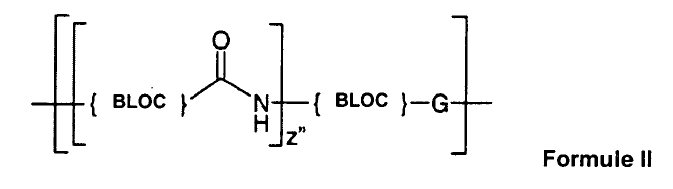 dans laquelle les groupements {BLOC} sont en outre, indépendamment l'un de l'autre, un groupement choisi parmi ceux de formule IIIA : 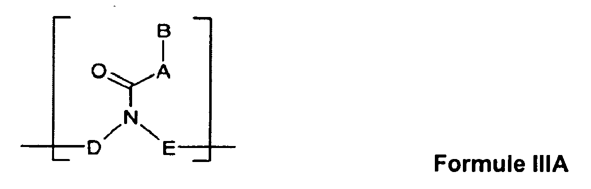 ou ceux de formule IIIB : 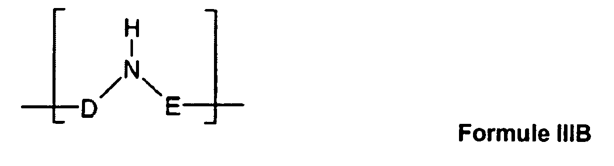 ou ceux de formules IV A à IV G : 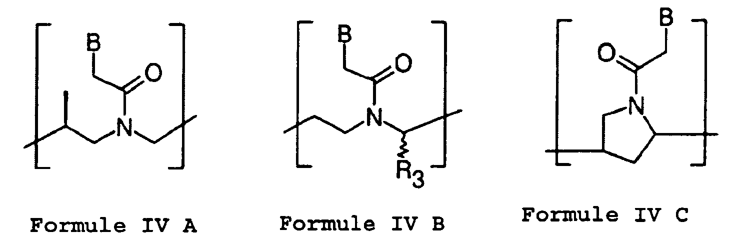 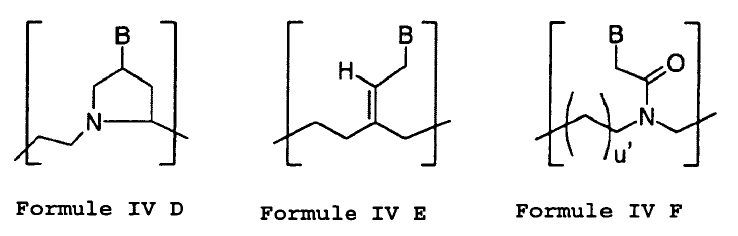 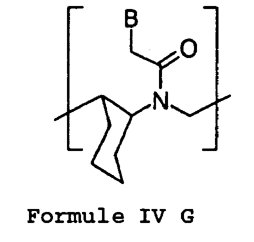 chaque composant {BLOC} pouvant être différent, et où, en outre,
z" vaut 0 à 100, de préférence 1 à 20, mieux encore 4 à 15,
le groupement G est choisi parmi les groupements (CR₅R₆)_{u'}, C(O)NH-(CR₁R₂)_{t'} ou C(O)NH-(CH₂CH₂O)_{u'}-CH₂CH₂, t' ayant une valeur de 2 à 10, de préférence 6,
les groupements A sont, indépendamment l'un de l'autre, un groupement (CR₁R₂)ₛ, où s vaut 1 à 3, de préférence 1,
les groupements B représentent, indépendamment l'un de l'autre, un radical aromatique, qui peut également posséder un caractère hétéroaromatique, ou de l'hydrogène, ou un groupement hydroxy ou alkyle en C₁₋C₁₈,
ou est une nucléobase courante dans la chimie des nucléotides, présente naturellement ou non, ou sa forme de promédicament,
les groupements D sont, indépendamment l'un de l'autre, un groupement (CR₃R₄)ₜ, t ayant une valeur de 2 à 10, de préférence de 2 à 4, mieux encore de 2,
les groupements E représentent, indépendamment l'un de l'autre un groupement (CR₅R₆)_{u'}, des radicaux adjacents R₅ et R₆ pouvant également former un cycle cycloalkyle en C₅-C₈ ou un composé spirannique,
les groupements R₅ et R₆ sont, indépendamment l'un de l'autre, un radical constitué de l'hydrogène ou d'un alkyle en C₁-C₆ ou le radical d'une chaîne latérale d'acide aminé, de préférence l'hydrogène, et où
u', R₁, R₂, R₃ et R₄ ont les mêmes significations que celles décrites précédemment,
ainsi que des sels physiologiquement acceptables du dérivé de PNA de formule I, sous réserve qu'au moins un radical T, T', Z ou Z' corresponde à un groupement hydroxy, mercapto, oxyanion ou thioate, et qu'au moins un radical B soit une nucléobase.

2. Dérivé de PNA selon la revendication 1, dans lequel au moins un radical T, T', Z ou Z' de la formule I représente un groupement oxyanion ou thioate dans une plage de pH de 4,5 à 14, de préférence de 6,5 à 12, mieux encore de 6,5 à 9.

3. Dérivé de PNA selon l'une quelconque des revendications 1 à 2, dans lequel n et m, indépendamment l'un de l'autre, sont égaux à 0.

4. Dérivé de PNA selon l'une quelconque des revendications 1 à 3, dans lequel q est égal à 1.

5. Dérivé de PNA selon l'une quelconque des revendications 1 à 4, dans lequel W et W' sont des groupements oxo.

6. Dérivé de PNA selon l'une quelconque des revendications 1 à 5, dans lequel T et Y' sont des groupements hydroxy ou oxyanion.

7. Dérivé de PNA selon l'une quelconque des revendications 1 à 6, dans lequel V et V' sont des groupements oxy.

8. Dérivé de PNA selon l'une quelconque des revendications 1 à 7, dans lequel X et X', indépendamment l'un de l'autre, sont un groupement U-(alcanediyle en C₂-C₂₂)-U, de préférence O-(alcanediyle en C₂-C₂₂)-O, mieux encore O-(CH₂)₂₋₆O, ou un groupement U-(CH₂CH₂-O)_{u'}, de préférence O(CH₂CH₂-O)_{u'}, où u' a une valeur de 1 à 6.

9. Dérivé de PNA selon l'une quelconque des revendications 1 à 8, dans lequel X, X', Z et Z' sont choisis, indépendamment l'un de l'autre, dans le groupe constitué de la fluorescéine, de la rhodamine, de la TAMRA ou d'un colorant de cyanine, de la biotine, du dabcyle, du psoralène, de l'acridine, du DNP, du cholestérol, de la vitamine E, du dabcyle, l'edans, de la lexitropsine, du psoralène, du BODIPY, du ROX, du R6G ou de la digoxygénine.

10. Dérivé de PNA selon l'une quelconque des revendications 1 à 9, dans lequel X, X', Z et Z' sont choisis, indépendamment l'un de l'autre, dans le groupe des monophosphates, des dérivés de la biotine et des dérivés de la fluorescéine.

11. Dérivé de PNA selon l'une quelconque des revendications 1 à 9, dans lequel Z est un marqueur de fluorescence et Z' un agent d'extinction.

12. Dérivé de PNA selon l'une quelconque des revendications 1 à 9, dans lequel Z est un agent d'extinction et Z' un marqueur de fluorescence.

13. Dérivé de PNA selon l'une quelconque des revendications 1 à 9, dans lequel Z et Z' sont, indépendamment l'un de l'autre, un radical alkyle en C₁₋C₂₂,
ou un radical U en C₁-C₂₂, de préférence un radical alcoxy en C₁-C₂₂, mieux encore un alcoxy en C₁₆,
ou un hydroxy-U en C₁-C₁₈, de préférence un hydroxy-C₁-C₁₈-O, mieux encore un HO-(CH₂)₃₋₁₂O,
ou un radical aminoalkyle-U, de préférence un radical aminoalcoxy, mieux encore un 6-aminohexoxy ou un 5-aminopentoxy,
ou un groupement de formule R₇-(CH₂CH₂-O)ₘ, dans laquelle R₇ est de préférence OH ou NH₂ et m vaut 1 à 6, mieux encore HO(CH₂CH₂-O)₂, HO(CH₂CH₂-O)₆ et H₂N-(CH₂CH₂₋O)₂,
ou un radical mercaptoalkyle-V, de préférence un radical mercaptoalcoxy, mieux encore un radical
6-mercaptohexyloxy.

14. Dérivé de PNA selon l'une quelconque des revendications 1 à 3, dans lequel q est égal à 0.

15. Dérivé de PNA selon la revendication 14, dans lequel D' est un acylamino, de préférence un acétylamino.

16. Dérivé de PNA selon l'une quelconque des revendications 1 à 15, dans lequel D est (CH₂)ₜ, de préférence (CH₂)₂.

17. Dérivé de PNA selon l'une quelconque des revendications 1 à 16, dans lequel A, E et G représentent CH₂.

18. Dérivé de PNA selon l'une quelconque des revendications 1 à 17, dans lequel B est une adénine, une cytosine, une 5-méthylcytosine, une guanine, une thymine et un uracile, ou une purine, la 2,6-diaminopurine, la N⁴N⁴-éthanocytosine, la N⁶N⁶-éthano-2,6-diaminopurine, le 5-alcynyl(C₃-C₆)-uracil, la 5-alcynyl (C₃-C₆)-cytosine, le 5-(1-propargylamino) - uracile, la 5-(1-propargylamino)-cytosine, la phénoxazine, la 9-aminoéthoxyphénoxazine, le 5-fluorouracile, une pseudoisocytosine, le 5-(hydroxyméthyl)uracile, le 5-aminouracile, un pseudouracile, un dihydrouracile, le 5-alkyl(C₁-C₆)-uracile, la 5-alkyl(C₁-C₆)-cytosine, la 5-alcényle(C₂₋C₆)-cytosine, la 5-fluorocytosine, le 5-chlorouracile, la 5-chlorocytosine, le 5-bromouracile, la 5-bromocytosine, la 7-désazaadénine, la 7-désazaguanine, la 8-azapurine ou une purine 7-désaza-7-substituée.

19. Dérivé de PNA selon l'une quelconque des revendications 1 à 18, dans lequel la séquence de bases est dirigée contre des parties de gènes suppresseurs de tumeur, des oncogènes ou des télomérases ou leurs produits de transcription.

20. Dérivé de PNA selon la revendication 19, dans lequel la séquence de bases de la partie PNA est dirigée contre l'initiation de la traduction de l'ARNm HA-ras.

21. Dérivé de PNA selon l'une quelconque des revendications 1 à 10 ou 13 à 20 pour une utilisation comme produit pharmaceutique.

22. Utilisation d'un dérivé de PNA selon l'une quelconque des revendications 1 à 10 ou 15 à 22 pour fabriquer un produit pharmaceutique pour la thérapie tumorale.

23. Dérivé de PNA selon l'une quelconque des revendications 1 à 20 pour une utilisation comme diagnostic.

24. Utilisation d'un dérivé de PNA selon l'une quelconque des revendications 1 à 20 pour détecter les microorganismes et/ou les virus.

25. Utilisation d'un dérivé de PNA selon l'une quelconque des revendications 1 à 20 pour détecter et/ou quantifier les acides nucléiques.

26. Utilisation d'un dérivé de PNA selon l'une quelconque des revendications 1 à 20 comme réactif de détection pour l'hybridation in situ ou l'hybridation in situ par fluorescence.

27. Utilisation d'un dérivé de PNA selon l'une quelconque des revendications 1 à 20 comme agent antisens, agent antigène, agent leurre ou agent de chiméroplastie.

28. Utilisation d'un dérivé de PNA selon l'une quelconque des revendications 11 ou 12 comme balise moléculaire.

29. Réactif de détection contenant un dérivé de PNA selon l'une quelconque des revendications 1 à 20.

30. Puce à PNA, contenant un dérivé de PNA selon l'une quelconque des revendications 1 à 20.

31. Biocapteur contenant un dérivé de PNA selon l'une quelconque des revendications 1 à 20.

32. Produit pharmaceutique, contenant un dérivé de PNA selon l'une quelconque des revendications 1 à 10 ou 15 à 22 et éventuellement d'autres additifs et/ou matériaux de support pharmacologiquement compatibles.

33. Agent antisens, agent antigène, agent leurre ou agent de chiméroplastie, contenant un dérivé de PNA selon l'une quelconque des revendications 1 à 20.

34. Procédé de fabrication d'un dérivé de PNA de formule I, dans laquelle
a) on relie l'extrémité C-terminale d'un acide nucléique amidé à un réactif de phosphorylation lié à une phase solide, ou on relie un acide nucléique amidé phosphorylé sur l'extrémité C-terminale à un support solide,
b) on prolonge l'ossature de l'oligomère de PNA par couplage séquentiel avec des monomères d'acide nucléique amidé, et
c) on le fait réagir éventuellement à l'extrémité N-terminale avec un réactif de phosphorylation.

35. Procédé selon la revendication 34, dans lequel on fabrique le PNA en utilisant les groupements protecteurs t-butyloxycarbonyle (BOC), 9-fluorénylméthoxycarbonyle (Fmoc) ou monométhoxytrityle (Mmt).

36. Procédé selon l'une quelconque des revendications 34 ou 35, dans lequel on fabrique le PNA en utilisant des supports solides.

37. Procédé selon la revendication 36, dans lequel on utilise comme support solide le CPG^{®}, le Tentagel^{®} ou l'aminométhylpolystyrène.

38. Procédé de fabrication d'un produit pharmaceutique, dans lequel
a) on fabrique un dérivé de PNA selon l'une quelconque des revendications 1 à 10 ou 15 à 20, et
b) on le fait réagir éventuellement avec d'autres additifs et/ou matériaux de support pharmacologiquement compatibles.

39. Procédé de fabrication d'une puce à PNA, dans lequel on fabrique d'abord un dérivé de PNA selon l'une quelconque des revendications 1 à 20, puis on le fixe sur un support solide, ou on fabrique le dérivé de PNA directement sur le support.

40. Procédé de fabrication d'un dérivé de PNA de formule I selon les revendications 34 à 37, **caractérisé en outre en ce que** l'on purifie le PNA en exploitant le caractère acide du radical phosphore par chromatographie ou électrophorèse.

41. Procédé selon la revendication 40, dans lequel on purifie le dérivé de PNA par chromatographie au moyen d'une phase stationnaire basique et d'un gradient d'un éluant acide ou contenant des sels.

42. Procédé selon la revendication 41, dans lequel la phase stationnaire est un échangeur d'anions ou une phase en mode mixte.
